# EUROPEAN PATENT APPLICATION

(11) **EP 4 648 060 A2**
(43) Date of publication of application: **12.11.2025**
(21) Application number: 25203569.6
(22) Date of filing: 06.03.2023
(51) Int. Cl.: G16H 40/63

(54) **METHOD OF QUALITY CONTROL, QUALITY CONTROL APPARATUS, AND PROGRAM**

(30) Priority: 09.03.2022 JP 2022035791; 09.03.2022 JP 2022035792
(62) Divisional of application: 23160154.3
(71) Applicant: SYSMEX CORPORATION, Kobe-shi Hyogo 651-0073 (JP)
(72) Inventor: Kawakami, Hajimu, Kobe-shi, Hyogo, 651-0073 (JP)
(74) Representative: Hoffmann Eitle

(57) **Abstract**

A method of quality control of a specimen prepared from a sample, includes: (S1) setting, from a control index information (MII) in which a plurality of indexes for the specimen are respectively associated with control values corresponding to the plurality of indexes, at least one index to be used for quality control of the specimen; (S3) obtaining a feature value relating to the at least one index from image data of the specimen; and (S5) outputting quality control information of the specimen based on the feature value and a control value associated with the at least one index.

## Description

### BACKGROUND

The present invention relates to a method of quality control, a quality control apparatus, and a program.

A method of executing quality control of a specimen preparing apparatus based on a staining state of a specimen is known. For example, in Patent Document 1, a system is disclosed that uses a specific color component of a nuclear region in a blood cell image of a smear as an index, compares a luminance value of the specific color component with a predetermined lower limit reference value and a predetermined upper limit reference value, and notifies an occurrence of staining abnormality when the luminance value is the predetermined lower limit reference value or less or the predetermined upper limit reference value or more.

Patent Document 1: Japanese Patent Application Publication No. 2010-169484

### SUMMARY

Smearing/staining conditions for a smear includes a reagent and an apparatus that are used to prepare the smear, and conditions of each process (e.g., a smearing condition, pH of a staining solution, temperature of the staining solution, and a staining time), and can affect the staining state of the smear. However, the smearing/staining conditions may differ from one testing facility to another, from one region or country to another, or from one reagent or apparatus that is used to another. Among such multiple smearing/staining conditions, there is a mearing/staining condition(s) that cannot properly perform quality control of the smear based on the index described in Patent Document 1.

A purpose of the present invention is to provide a method of quality control, a quality control apparatus, and a program that are capable of performing quality control of a smear appropriately under smearing/staining conditions adopted in each facility.

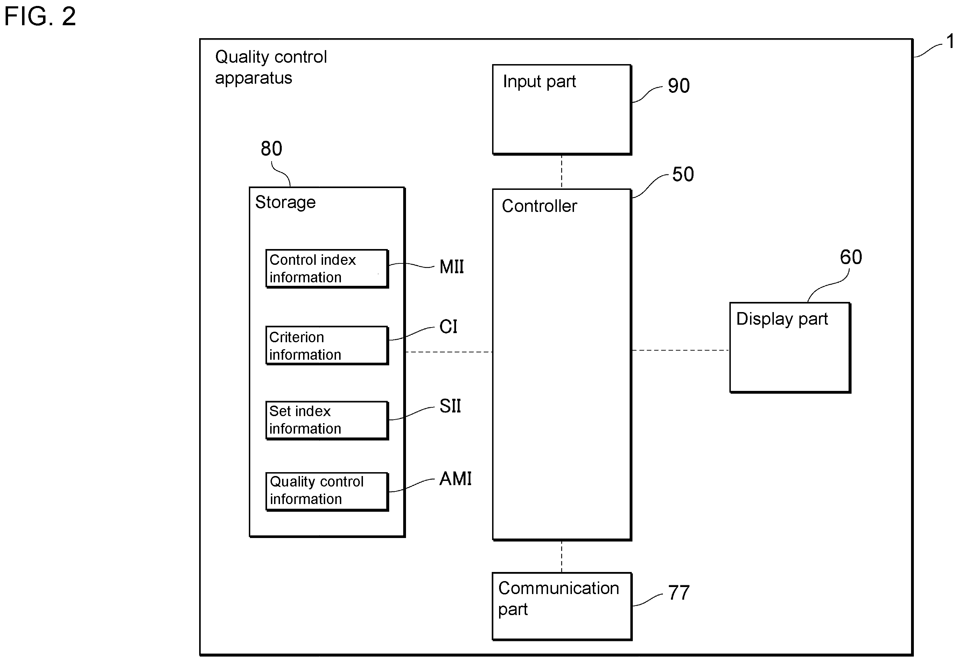

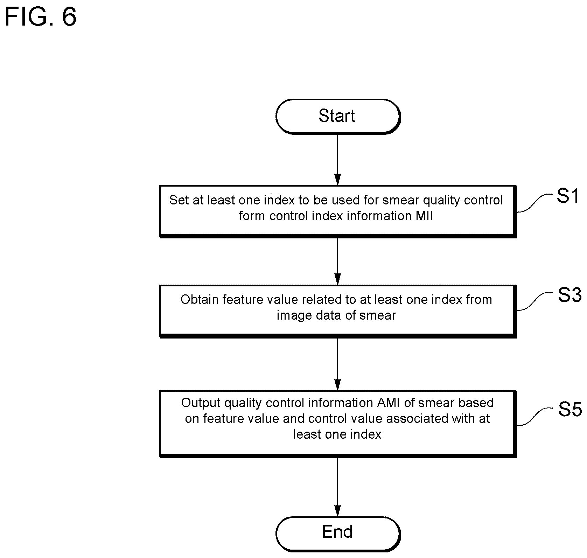

As illustrated in FIG. 2 and FIG. 6, a method of quality control according to the present invention is a method of quality control of a specimen prepared from a sample that is characterized by including: (S1) setting, from a control index information (MII) in which a plurality of indexes for the specimen are respectively associated with control values corresponding to the plurality of indexes, at least one index to be used for the quality control for the specimen; (S3) obtaining, from image data of the specimen, a feature value related to the at least one index; and (S5) outputting, based on the feature value and the control value that is associated with the at least one index, quality control information for the specimen.

According to the quality control method of the present invention, at least one index to be used for the quality control for the specimen is set from the control index information (MII), a feature value related to the at least one index is obtained from image data of the specimen, and quality control information for the specimen is output based on the feature value and the control value that is associated with the at least one index. Thus, the quality control method according to the present invention can appropriately perform the specimen quality control under smearing/staining conditions adopted by each facility.

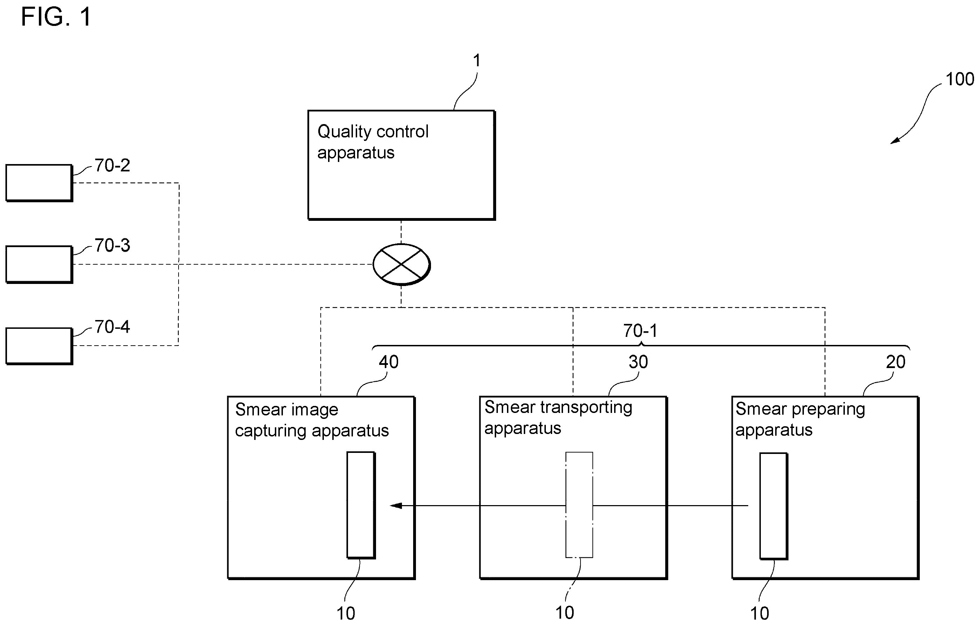

As illustrated in FIG. 1, FIG. 2, and FIG. 6, a quality control apparatus (1) according to the present invention is a quality control system to perform quality control of a specimen prepared from a sample that is characterizing by including a controller (50), wherein the controller (50) is configured to: set, from a control index information (MII) in which a plurality of indexes for the specimen are respectively associated with control values corresponding to the plurality of indexes, at least one index to be used for the quality control for the specimen; obtain a feature value related to the at least one index from image data of the specimen; and output, based on the feature value and the control value associated with the at least one index, quality control information for the specimen.

According to the quality control apparatus (1) of the present invention, the controller (50) sets at least one index to be used for the specimen quality control from the control index information (MII), and obtains a feature value related to the at least one index from the image data of the specimen. The controller (50) outputs quality control information for the specimen based on the feature value and the control value that is associated with the at least one index. Thus, the quality control system according to the present invention can appropriately perform the specimen quality control under smearing/staining conditions adopted in each facility.

As illustrated in FIG. 1, FIG. 2, and FIG. 6, a program according to the present invention is a program that causes a quality control apparatus (1) that is configured to perform quality control for a specimen prepared from a sample to execute: (S1) setting, from a control index information (MII) in which a plurality of indexes for the specimen are respectively associated with control values corresponding to the plurality of indexes, at least one index to be used for the quality control for the specimen; (S3)obtaining a feature value related to the at least one index from the image data of the specimen; and (S5) outputting, based on the feature value and the control value that is associated with the at least one index, quality control information for the specimen.

According to the program of the present invention, at least one index to be used for the specimen quality control is set from the control index information (MII), a feature value related to the at least one index is obtained from image data of the specimen, and quality control information for the specimen is output based on the feature value and the control value that is associated with the at least one index. Thus, the program according to the present invention can appropriately perform the specimen quality control under smearing/staining conditions adopted by each facility.

According to the present invention, it is possible to appropriately perform specimen quality control under smearing/staining conditions adopted by each facility.

### BRIEF DESCRIPTION OF DRAWINGS

[FIG. 1] FIG. 1 is a schematic diagram illustrating an overview of a quality control system.
[FIG. 2] FIG. 2 is a block diagram illustrating an example of a configuration of a quality control apparatus.
[FIG. 3A] FIG. 3A is a diagram illustrating an overview of control index information.
[FIG. 3B] FIG. 3B is a diagram illustrating an overview of set index information.
[FIG. 4] FIG. 4 is a diagram illustrating an overview of a process of obtaining a feature value.
[FIG. 5] FIG. 5 is a diagram illustrating an overview of an output screen of quality control information.
[FIG. 6] FIG. 6 is a flowchart illustrating an example of an output control process of quality control information for a specimen.
[FIG. 7] FIG. 7 is a flowchart illustrating a first example of an index setting process.
[FIG. 8] FIG. 8 is a diagram illustrating an example of a setting screen for user selection in a first example of the index setting process.
[FIG. 9] FIG. 9 is a diagram illustrating an example of a setting screen for file selection in a first example of the index setting process.
[FIG. 10] FIG. 10 is a flowchart illustrating a second example of an index setting process.
[FIG. 11] FIG. 11 is a diagram illustrating an example of a process using a Region ID in a second example of the index setting process.
[FIG. 12] FIG. 12 is a diagram illustrating an example of a process using Guideline ID in a second example of the index setting process.
[FIG. 13] is a diagram illustrating another example of a process in a second example of the index setting process.
[FIG. 14] FIG. 14 is a diagram illustrating an example of a setting screen in a second example of the index setting process.
[FIG. 15] FIG. 15 is a diagram illustrating an example of a process using a Vender Recommend ID in a second example of the index setting process.
[FIG. 16] FIG. 16 is a diagram illustrating an example of a process using a preparing condition ID in a second example of the index setting process.
[FIG. 17] FIG. 17 is a diagram illustrating an example of a setting screen for condition selection in a second example of the index setting process.
[FIG. 18] FIG. 18 is a flowchart illustrating a third example of an index setting process.
[FIG. 19] FIG. 19 is a diagram illustrating an example of an information process of a control center and a testing system in a third example of the index setting process.
[FIG. 20] FIG. 20 is a flowchart illustrating a process of obtaining a feature value that reflects a staining state of a smear, which is an object to be controlled.
[FIG. 21] FIG. 21 is a flowchart illustrating an example of a process of obtaining a feature value.
[FIG. 22] FIG. 22 is a diagram illustrating an example of a process of obtaining a feature value.
[FIG. 23] FIG. 23 is a diagram illustrating examples of feature values that reflect a staining state of red blood cells.
[FIG. 24] FIG. 24 is a diagram illustrating an example of a process of obtaining a mean value of luminance values of red components of red blood cells.
[FIG. 25] FIG. 25 is a diagram illustrating examples of feature values that reflect stainability of white blood cells.
[FIG. 26] FIG. 26 is a diagram illustrating examples of feature values that reflect forms of blood cells.
[FIG. 27] FIG. 27 is a flowchart illustrating an example of a process of obtaining a granule index.
[FIG. 28] FIG. 28 is a diagram illustrating an example of a process of obtaining a granule index.
[FIG. 29] FIG. 29 is a diagram illustrating an example of a process of extracting primary granules and secondary granules.
[FIG. 30] FIG. 30 is a diagram illustrating an example of an output screen of feature values reflecting staining states of smears to be controlled and quality control information.
[FIG. 31] FIG. 31 is a diagram illustrating an example of an output screen of feature values reflecting staining states of smears to be controlled and quality control information.
[FIG. 32] FIG. 32 is a diagram illustrating an example of an output screen of feature values reflecting staining states of smears to be controlled and quality control information.
[FIG. 33] FIG. 33 is a diagram illustrating an example of a screen displaying a plurality of image data that are obtained from a smear.
[FIG. 34] FIG. 34 is a diagram illustrating an example of a screen displaying specific image data in association with a plurality of feature values that are associated with the specific image data.
[FIG. 35] FIG. 35 is a diagram illustrating an example of a screen from which a feature value(s) to be obtained can be selected.
[FIG. 36] FIG. 36 is a diagram illustrating an example of a screen on which a normal range can be set for one of feature values.

### DETAILED DESCRIPTION

Preferred embodiments of the present invention are described with reference to the drawings below. The same symbols are attached to the same elements, and duplicate descriptions are omitted. Also, the positional relationships such as up, down, left, right, etc. are based on the positional relationships illustrated in the drawings, unless otherwise specified. Furthermore, the dimensional ratios in the drawings are not limited to the ratios illustrated in the figures. Moreover, the following embodiments are examples to illustrate the present invention, and the present invention is not limited to these embodiments.

### (Overview of quality control system)

Referring to FIG. 1 and FIG. 2, an overview of a quality control system 100 is described that is configured to output quality control information of a smear (a smear specimen) of blood collected from a subject. FIG. 1 is a schematic diagram illustrating the overview of the quality control system 100.

The quality control system 100 includes a plurality of testing systems 70-1 to 70-4 each including a smear preparing apparatus 20, a smear transporting apparatus 30, and a smear image capturing apparatus 40, and a quality control apparatus 1 that outputs quality control information.

Each of the testing systems 70-1 to 70-4 is a system that prepares smear slides 10 by the smear preparing apparatus 20, transports the prepared smear slides 10 to the smear image capturing apparatus 40 by the smear transporting apparatus 30, captures image of the prepared smear specimen slides 10 by the smear image capturing apparatus 40, obtains image data of smears, and transmits the image data to the quality control apparatus 1. The testing systems 70-1 to 70-4 are installed in multiple testing facilities (testing facilities A to D). The multiple testing facilities (testing facilities A to D) differ from each other in smearing/staining conditions when preparing smears. The quality control apparatus 1 is installed at a facility of a provider of the smear image capturing apparatus 40 (e.g., a manufacturer of the smear image capturing apparatus 40) and is connected via a network to the smear preparing apparatus 20, the smear transporting apparatus 30, and the smear image capturing apparatus 40 of each of the testing systems 70-1 to 70-4.

FIG. 2 is a block diagram illustrating an example of a configuration of the quality control apparatus 1. As illustrated in FIG. 2, the quality control apparatus 80 includes a controller 50, a storage 80, an input part 90, a display part 60, and a communication part 77.

The controller 50 includes a CPU that, for example, executes information processing to output smear quality control information. Also, the controller 50 is capable of communicating with the smear preparing apparatus 20, the smear transporting apparatus 30, and the smear image capturing apparatus 40 illustrated in FIG. 1 via the communication part 77. The storage 80 includes a memory that stores, for example, information for executing information processing of the controller 50 and information generated by executing the information processing. The input part 90 is, for example, a keyboard, a mouse, or the like and the display part 60 is, for example, a liquid crystal display, an organic EL display, or the like.

The storage 80 stores, for example, control index information MII, a criterion information Cl, set index information SII, and quality control information AMI. The "criterion information Cl" is information related to predetermined criteria for setting indexes to be used in the quality control. The predetermined criteria include, for example, guidelines for the smear quality control that are established for a given country, region, or organization. The predetermined criteria include criteria for setting indexes that is defined according to a condition for preparing a smear.

The storage 80 stores the control index information MII in which a plurality of indexes for a smear are respectively associated with control values corresponding to the plurality of indexes, and the set index information SII which indicates an index(es) to be used for the smear quality control. For each of the testing facilities A to D, the quality control apparatus 1 sets, from the plurality of indexes in the control index information MII stored in the storage 80, at least one index suitable for the quality control as the set index information SII. The quality control apparatus 1 receives image data of the smear from the smear image capturing apparatus 40 via the network. The quality control apparatus 1 obtains, from the received image data, a feature value that relates to each of the set index(es), and outputs quality control information AMI for the smear based on the obtained feature value(s) and a control value(s) that is associated with the set index(es).

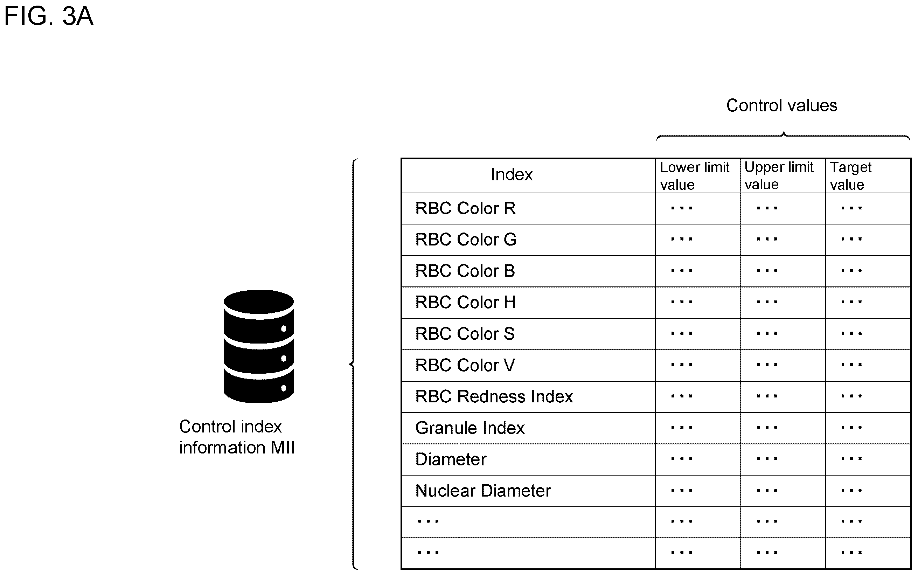

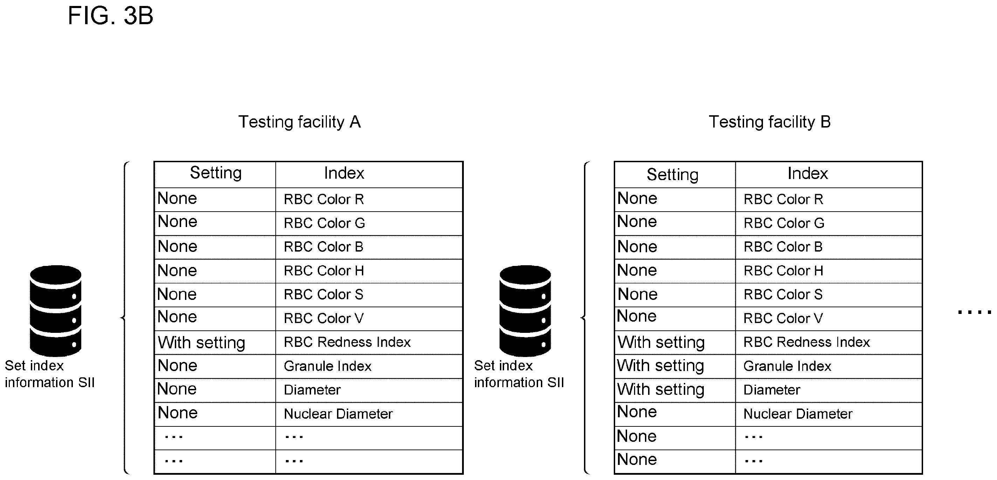

Referring to FIGS. 3A and 3B, control index information MII in which a plurality of indexes for a smear and control values corresponding to each of the plurality of indexes are associated, and set index information SII which indicates index(es) to be used for smear quality control are explained.

FIG. 3A is a diagram illustrating an overview of the control index information MII stored in the storage 80 illustrated in FIG. 2. As illustrated in FIG. 3A, the control index information MII is a database, in which the plurality of indexes for the smear and the control values corresponding to each of the plurality of indexes (e.g., a lower limit value, an upper limit value, and a target value) are associated. The plurality of indexes regarding the smear are control items that reflect a staining state of the smear and a form of a specific cell in the smear slide 10. The plurality of indexes regarding the smear includes, for example, at least color information and/or form information of a blood cells in the blood. In the control index information MII, RBC Color R (luminance value of a red component of the red blood cells), RBC Color G (luminance value of a green component of the red blood cells), RBC Color B (luminance value of a blue component of the red blood cells), RBC Color H (hue value of the red blood cells), RBC Color S (saturation value of the red component of the red blood cells), RBC Color V (brightness value of the red component of the red blood cells), RBC Redness Index (redness index of the red blood cells), Granule Index (granule index of the white blood cells), Diameter (cell diameter of the blood cells), Nuclear Diameter (nuclear diameter of the blood cells), etc. are stored as the indexes.

FIG. 3B is a diagram illustrating an overview of the set index information SII stored in the storage 80 illustrated in FIG. 2. As illustrated in FIG. 3B, the set index information SII is a database storing therein an index(es) to be used for the smear quality control. In the set index information SII, indexes corresponding to the plurality of indexes stored in the control index information MII are associated with settings that indicate whether or not each of the indexes is to be used for the quality control. For example, in the set index information SII for a testing facility A, the index "RBC Redness Index" is set as an index to be used. In the set index information SII for a testing facility B, the index "RBC Redness Index", the index "Granule Index", and the index "Diameter" are set as indexes to be used. Similarly, the set index information SII for each of the testing facilities C and D is stored in the storage 80.

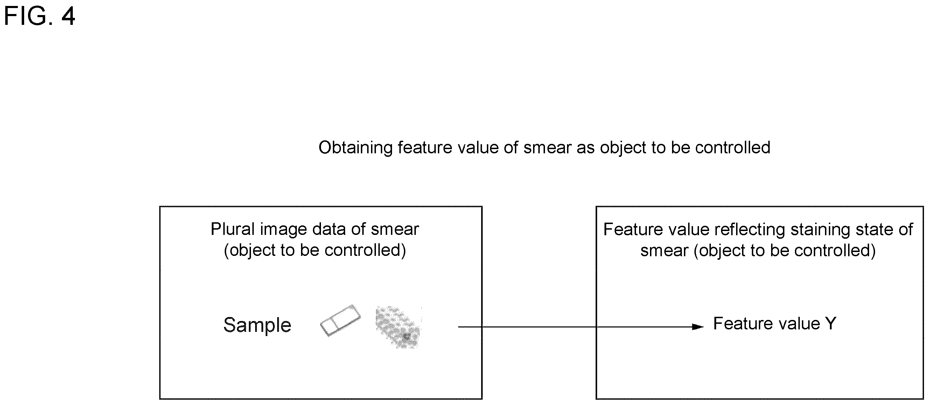

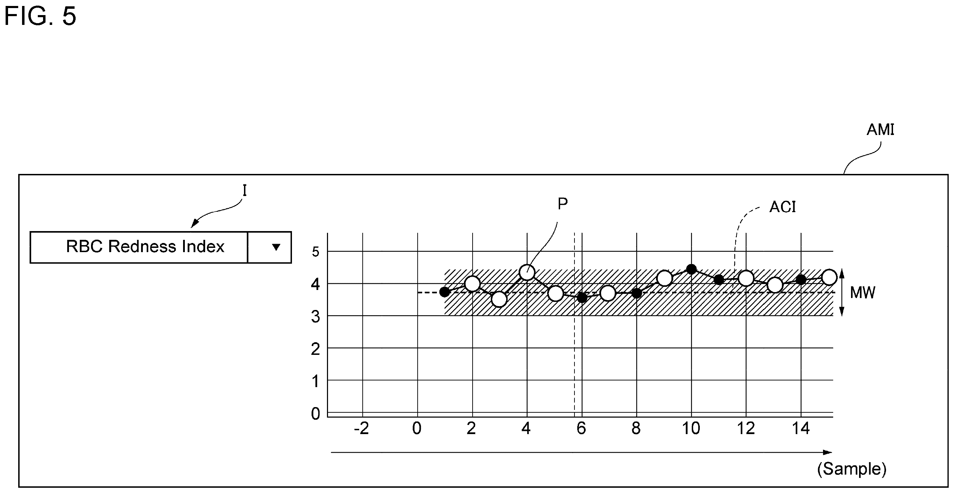

With reference to FIG. 4 and FIG. 5, a process of obtaining a feature value of a smear which is an object to be controlled and a process of outputting quality control information are described.

FIG. 4 is a diagram illustrating an overview of a process of obtaining a feature value of a smear to be controlled.

As illustrated in FIG. 4, in a process of obtaining a feature value Y, related to the index that is set at the set index information SII illustrated in FIG. 3B, of a smear to be controlled, the quality control apparatus 1 obtains image data of regions of blood cells in the blood from the smear to be controlled (e.g., Sample), and obtains the feature value Y that reflects a staining state of the smear to be controlled from the obtained image data. The feature value Y that reflects the staining state of the smear to be controlled is obtained from the image data of at least one smear out of all smears taken on that day, for example.

FIG. 5 is a diagram illustrating an overview of an output screen of quality control information. A quality control information AMI displays an index(es) that is set in the set index information SII and does not display indexes that are not set in the set index information SII. An example illustrated in FIG. 5 is an example of outputting the smear quality control information AMI, based on control values that are associated with an index I set in the set index information SII for one testing facility (testing facility A) and the feature value Y related to the index I. For example, the quality control information AMI is a diagram (e.g., a chart or a graph) or notification information (e.g., an alert) that displays the control values associated with the index I and the feature value Y of each smear in a comparable manner. The example illustrated in FIG. 5 is a chart that outputs the control values associated with the index I set as the set index information SII for the testing facility (testing facility A) and the feature value Y related to the index I that reflects the staining state of each smear to be controlled. The control values associated with the index I is generated on a daily basis from the image data of all smears taken on that day, for example.

As illustrated in FIG. 5, the control values associated with the index I that is set as the set index information SII for the one testing facility (testing facility A) and a plurality of plots P of the feature values Y regarding to the index I that reflect the staining states of the smears to be controlled are output. For example, as the control values, a target value ACI for the index I and a control width MW for the index I are output. By visually understanding the relationship between the output plots P and the control values including the target value ACI and the control width MW, the user can easily perform quality control on the smears to be controlled. For example, by outputting, in comparison with the target ACI and the control width MW which are the control values with respect to the index I that is set as the set index information SII for the one testing facility (testing facility A), the plots P of the feature values Y of the smears to be controlled with respect to the index I, the laboratory technician of the testing facility A can perform the quality control in the smears to be controlled. Here, a horizontal axis ("Sample") in the output chart indicates the smears to be controlled, for example, a smear of Sample 2 and a smear of Sample 4 are different smears. A vertical axis indicates a magnitude of the feature value that reflects the staining state of each smear to be controlled.

A control value stored in the control index information MII is a median or a mean value that is set for each of the plurality of indexes. Also, a control value includes at least one of an upper limit value and a lower limit value that are, for example, ±2 SD (Standard Deviation: standard deviation) or ±3 SD with respect to the median or the mean value. A control value may include, for example, a value based on a moving average, other than the median and the mean value. Also, a control value may be only the median or the mean value.

As a process of generating control values to be stored in the control index information MII, the control values are generated by obtaining image data of respective regions of blood cells in blood from each of a plurality of smears, obtaining feature values reflecting the staining states of the plurality of smears from the plurality of image data, and thereby obtaining the control values, for example, including a target value and a control width defined by an upper limit value and a lower limit value. The target value is calculated from the mean value of the obtained plural feature values, and the control width defined by the upper limit value and the lower limit value is calculated from ±2 SD (Standard Deviation: standard deviation) with respect to the target value.

The feature value is quantified information that reflects the staining state of the smear in the image data of the regions of the blood cells in the blood. Also, the feature value include, for example, color information of red blood cells, and is generated by obtaining a color index of an intracellular region for each cell from each of the plurality of image data and by quantifying the staining state in the smear of each of the plurality of image data.

The quality control information AMI is information, for example, in which an index(es) for smears, a control value(s) corresponding to the index(es), and a feature value(es) concerning the index(es) obtained from image data are associated.

By outputting the quality control information AMI, it is possible to compare the control values with the feature values regarding the set index(es), so as to understand whether there is a problem with the staining state of the smear to be controlled, for example. In such cases, it is possible to ensure the quality of the controlled smear based on the quality control information AMI.

The timing of outputting the quality control information AMI is arbitrary and may be, for example, every day, every few days, every week, or every month, or every few hours.

Test subjects are primarily humans, but may be other animals other than humans. The quality control system 100 performs a clinical testing or an analysis for medical research, for example, of a sample collected from a patient. The sample is a sample of biological origin. The sample of biological origin is, for example, a liquid such as blood (whole blood, serum, or plasma), urine, or other body fluid collected from a test subject, or a liquid obtained by performing a specified pretreatment on the collected body fluid or blood. The sample may also be, for example, a piece of tissue or a cell of the subject, other than a liquid.

The smear preparing apparatus 20 is an apparatus configured to perform a smearing process of smearing a sample on a slide and a staining process of staining the sample onto the slide 10. The smear preparing apparatus 20 aspirates a specimen as the sample, drops and smears the specimen onto the slide, and stains the specimen to thereby obtain the smear slide 10.

The smear transporting apparatus 30 is configured to receive the smear slide 10 prepared by the smear preparing apparatus 20 and transport the smear slide 10 to the smear image capturing apparatus 40. The smear transporting apparatus 30 also is configured, after the smear slide 10 is imaged by the smear image capturing apparatus 40, to receive and store the smear slide 10.

The smear image capturing apparatus 40 is configured capture the image of the smear slide 10 that is transported by the smear transporting apparatus 30.

The testing system 70-1 to 70-4 may include other apparatuses. For example, the testing system 70-1 to 70-4 may include an analyzer that performs analysis of a sample (e.g., a hematology analyzer that executes classification and counting of blood cells in a sample) and a transporting apparatus that transports a container in which a sample is contained.

The testing system 70 including the smear preparing apparatus 20, the smear transporting apparatus 30, and the smear image capturing apparatus 40 is described, for example, in the specification of U.S. Patent Application Publication No. 2019/0049474. The specification of U.S. Patent Application Publication No. 2019/0049474 is hereby incorporated herein by reference.

FIG. 6 is a flowchart illustrating an example of an output control process of the smear quality control information. As illustrated in FIG. 6, the controller 50 of the quality control apparatus 1 illustrated in FIG. 2 sets, from the control index information MII stored in the storage 80, at least one index to be used for the smear quality control (step S1). The controller 50 obtains a feature value relating to the at least one index from the image data of the smear (step S3). The controller 50 outputs the smear quality control information AMI based on the feature value and the control value associated with the at least one index to the display part 60 (step S5). In the following, a specific example of step S1 for an index setting process is described.

### (First example of index setting process)

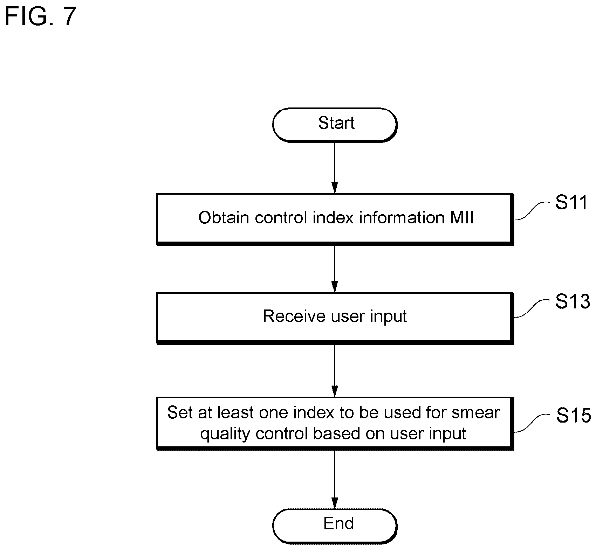

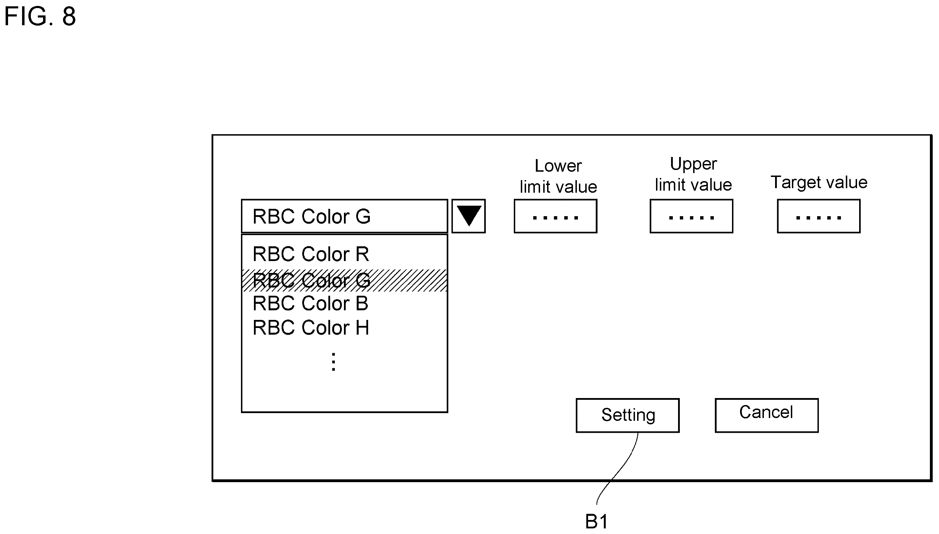

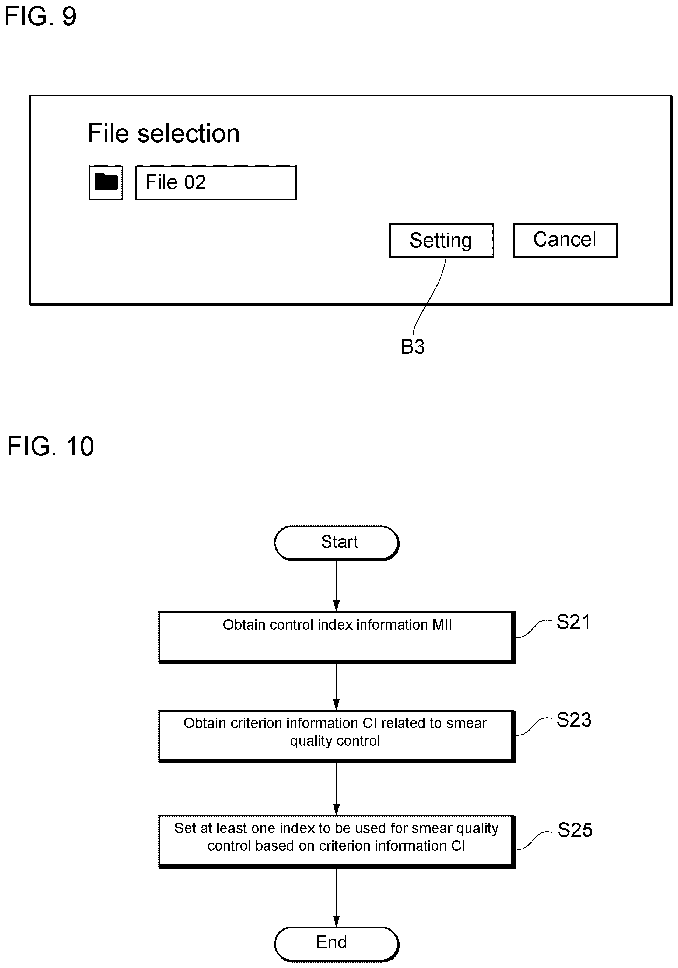

In a first example of an index setting process, the controller 50 of the quality control apparatus 1 sets an index(es) based on a user input via the input part 90. FIG. 7 is a flowchart illustrating the first example of the index setting process. The controller 50 obtains the control index information MII from the storage 80 illustrated in FIG. 2 (step S11). The controller 50 receives a user input via the input part 90 (step S13). The controller 50 sets at least one index to be used for the smear quality control based on the user input via the input part 90 (step S15). In the first example of the index setting process, the index(es) and the control value(s) to be used for the quality control are, for example, set by the user selecting, from the plurality of indexes in the control index information MII stored in the storage 80, the index(es) and the control value(s) via the input part 90. With reference to FIG. 8 and FIG. 9, examples of setting screens in the first example of the index setting process are described.

FIG. 8 is a diagram illustrating an example of a setting screen for user selection in the first example of the index setting process. As illustrated in FIG. 8, the user optionally selects an index (e.g., "RBC Color G") from the plurality of indexes included in the control index information MII, so that the selected index is set. For example, when the index is selected by the user, the control values including the lower limit value, the upper limit value, and the target value associated with the selected index are automatically extracted from the control index information MII. Note that each of the extracted control values may be set to be changeable by the user. When a setting button B1 is selected by the user, the index and the control values associated with the index, which are to be used for the quality control, are recorded as the set index information SII illustrated in FIG. 2.

FIG. 9 is a diagram illustrating an example of a setting screen for file selection in the first example of the index setting process. First, created index file data (e.g., CSV files) are displayed on the screen for selection. Each of the index file data includes information corresponding to at least one index to be used in the quality control and a control value(s) corresponding to the index, based on the control index information MII. As illustrated in FIG. 9, when the user selects a specific index file (e.g., "file 02") and presses a setting button B3, the selected index file data is imported so as to set the set index information SII based on the information included in the imported index file data.

### (Second example of index setting process)

In a second example of an index setting process, the controller 50 of the quality control apparatus 1 sets an index(es) based on a criterion information CI. FIG. 10 is a flowchart illustrating the second example of the index setting process. The controller 50 obtains the control index information MII from the storage 80 illustrated in FIG. 2 (step S21). The controller 50 obtains the criterion information CI for the smear quality control from the storage 80 based on a user input via the input part 90 (step S23). The controller 50 sets at least one index to be used for the smear quality control based on the user input via the input part 90 (step S25).

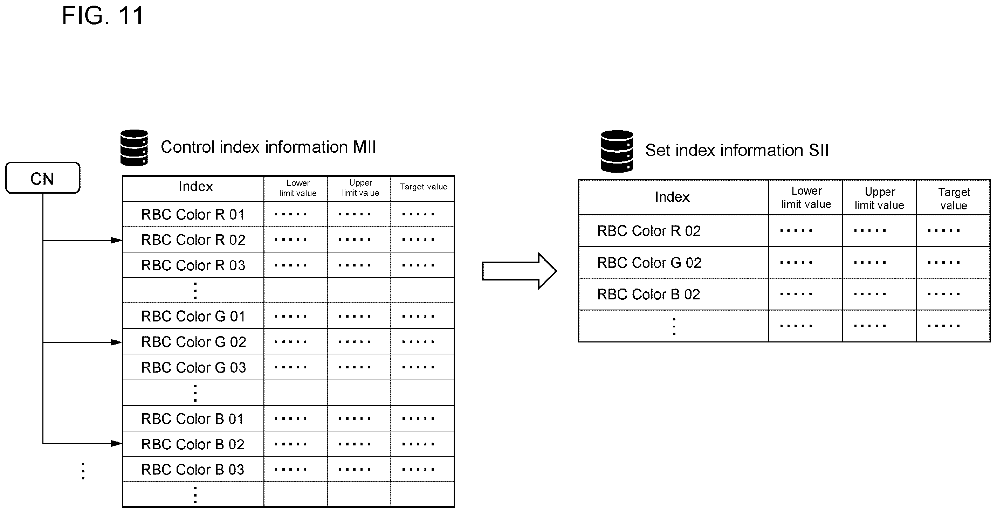

Referring to FIGS. 11 to 14, methods of setting an index(es) to be used in the quality control from the control index information MII based on guidelines for the smear quality control established for a given country, region, or organization is described. In such a method, each index or each control value in the control index information MII is associated with at least one of region type information, such as a country or region where a testing facility is located or an organization to which the testing facility belongs, and guideline type information applied according to the location or affiliation of the testing facility.

FIG. 11 is a diagram illustrating an example of the second example of the index setting process using Region ID. Region ID is an example of the region type information, such as the country or region where the testing facility is located or the organization to which the testing facility belongs. As illustrated in FIG. 11, the controller 50 of the quality control apparatus 1 specifies, for example, Region ID "CN" which indicates China, based on a user input via the input part 90 illustrated in FIG. 2. Then, the controller 50 extracts one or more indexes corresponding to Region ID "CN" and control values associated with each of the one or more indexes from the control index information MII stored in the storage 80, so as to set them as the set index information SII. Note that each region type information is associated with respective indexes in the control index information MII based on the guideline information defined for each country, region, or organization.

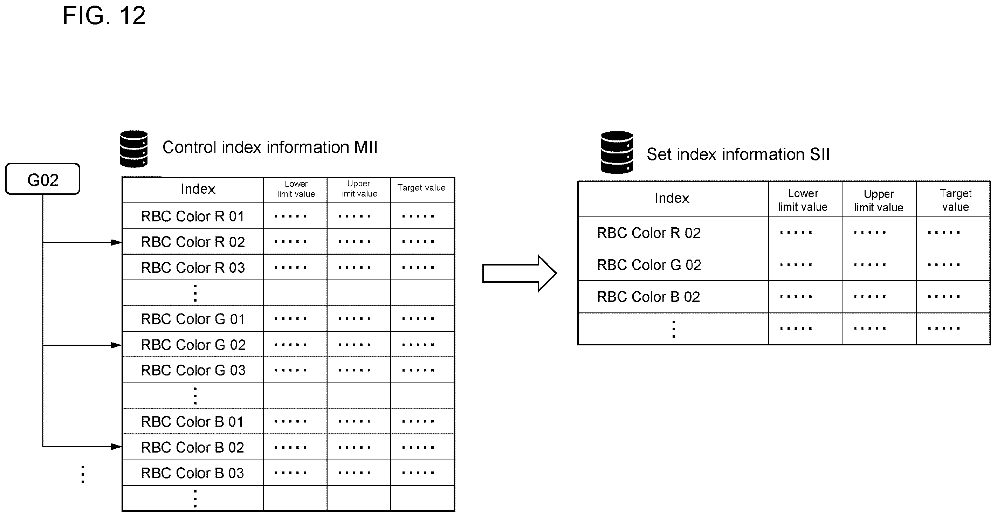

FIG. 12 is a diagram illustrating the second example of the index setting process using Guideline ID. Guideline ID is an example of the guideline type information that is applied according to the location and affiliation of the testing facility. As illustrated in FIG. 12, the controller 50 of the quality control apparatus 1 specifies, for example, Guideline ID "G02" which indicates a Chinese guideline, based on a user input via the input part 90. Then, the controller 50 extracts one or more indexes associated with Guideline ID "G02" from the control index information MII stored in the storage 80 and control values corresponding to each of the one or more indexes, so as to set them as the set index information SII. Note that each guideline type information is associated with respective indexes in the control index information MII based on the guideline information defined in each country, region, or organization.

In the examples of FIG. 11 and FIG. 12, a different control value(s) may be set for the same type of index depending on the region type information or the guideline type information. For example, regarding the index "RBC Color R" in FIG. 11 and FIG. 12, the upper, lower, and target values set corresponding to "RBC Color R 01", "RBC Color R 02", "RBC Color R 03", etc., may differ from each other depending on the region type information or the guideline type information.

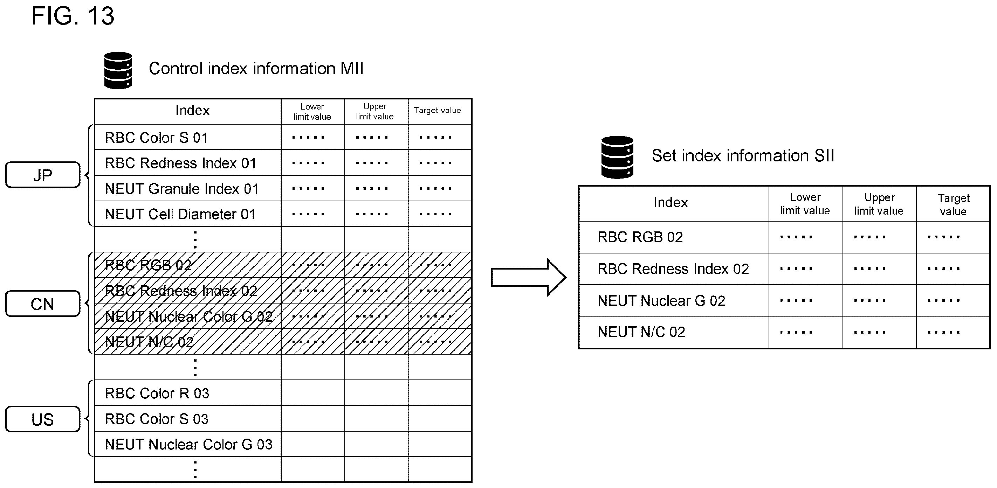

Also, FIG. 13 illustrated an example of setting indexes and control values corresponding to Region ID "CN" which indicates China. As illustrated in FIG. 13, different types of indexes for the quality control may be set according to the region type information or the guideline type information. FIG. 13 is an example in which different types of indexes are set according to the region type information. For example, the types of indexes associated with Region ID "JP" for Japan (RBC Color S, RBC Redness Index, NEUT Granule Index, NEUT Cell Diameter) includes at least one different from the types of indexes associated with Region ID "CN" for China (RBC RGB, RBC Redness Index, NEUT Nuclear Color G, and NEUT N/C). When Region ID "CN" indicating China is specified, the indexes associated with "CN" and the control values associated with each of the indexes are extracted and set as the set index information SII.

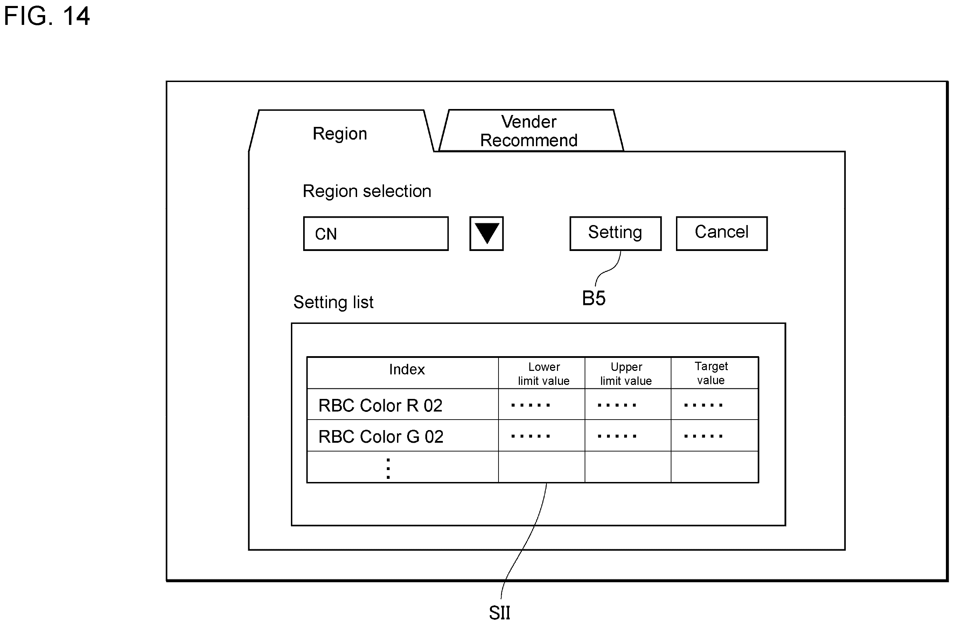

FIG. 14 is a diagram illustrating an example of a setting screen in the second example of the index setting process. First, on the screen, multiple Region IDs, such as "JP", "US", or "CN", are displayed in a pull-down format for selection. As illustrated in FIG. 14, when the user selects Region ID (e.g., "CN") and press a setting button B5, one or more indexes associated with Region ID "CN" and control values associated with each of the one or more indexes are extracted from the control index information MII and set as the set index information SII. The information of the set indexes and control values are displayed in a setting list.

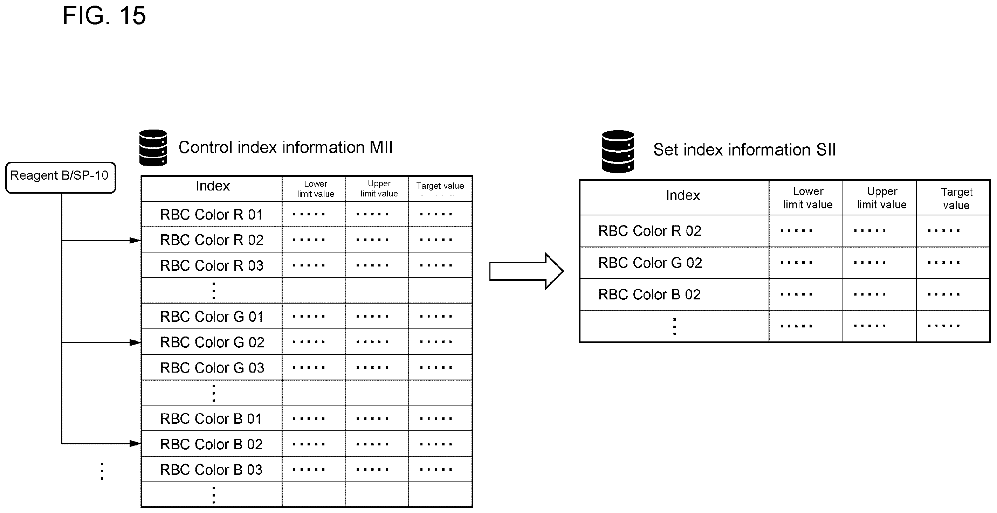

Next, referring to FIG. 15, a method of setting, based on a predetermined standard determined according to a smear preparation condition, indexes to be used in the quality control from the control index information MII is described. The predetermined standard according to the smear preparation condition is information that defines indexes to be used for the quality control and control values thereof according to the type of a reagent used for preparing a smear and/or the type of a smear preparing apparatus. Such information can be provided to the user, for example, as information on a recommended quality control condition from a provider of a reagent or an apparatus used to prepare a smear or a provider of a software of an analysis or quality control. In the method, the indexes and control values included in the control index information MII are associated with at least one of the following: information that relates to the type of a reagent used to prepare a smear and/or the type of a smear preparing apparatus (Vendor Recommend ID), and information that relates to the type of a preparing condition set according to the reagent and/or the apparatus to prepare a smear (Preparing Condition ID).

Here, the "reagent type" (e.g., "reagent A") is information about, for example, a staining solution or buffer solution, or a combination thereof. Especially pH affects a staining state of a smear, and thus an important parameter. The "type of a smear preparing apparatus" is, for example, information about the type of the smear preparing apparatus or setting information thereon. The staining state of the smear is particularly affected by a staining condition and staining time, and a cell diameter is particularly affected by a drying condition (e.g., forced drying or natural drying).

FIG. 15 is a diagram illustrating an example of a process using information (Vender Recommend ID) on the type of the reagent used to prepare a smear and/or the type of a smear preparing apparatus in the second example of the index setting process. As illustrated in FIG. 15, the controller 50 of the quality control apparatus 1 specifies, from a plurality of Vender IDs, for example, "Reagent A/SP-10", "Reagent B/SP-10", "Reagent A/SP-50", and the like, a specific Vender Recommend ID "Reagent B/SP-10". Then, the controller 50 extracts one or more indexes that are associated with Vender Recommend ID "Reagent B/SP- 10" and control values associated with each of the indexes from the control index information MII stored in the storage 80, so as to set the indexes and the control values as the set index information SII. Note that the indexes included in the control index information MII are associated with the Vender Recommend ID based on a predetermined standard determined according to the smear preparing condition.

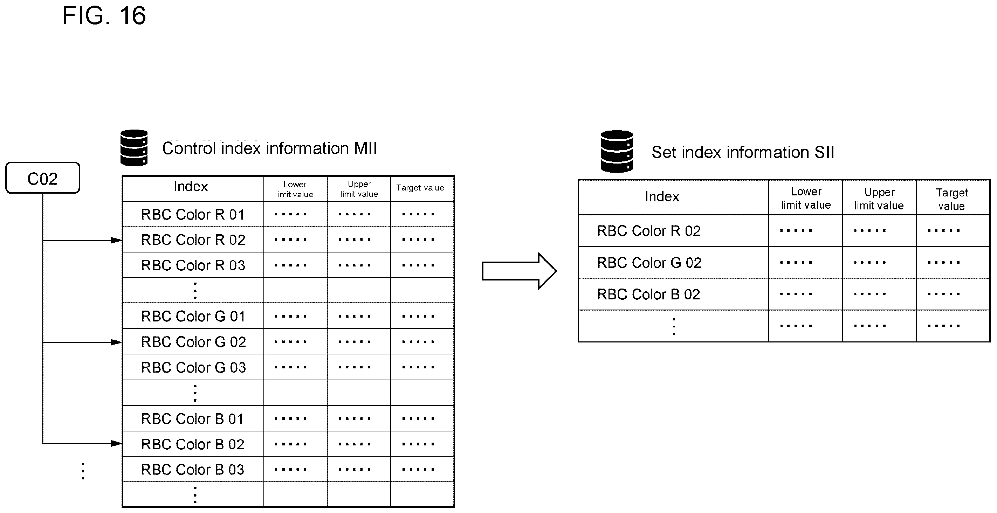

Next, referring to FIG. 16, a method of setting, based on a predetermined standard determined according to a smear preparing condition, indexes to be used in the quality control from the control index information is described.

FIG. 16 is a diagram illustrating an example of a process using the type information of the preparing condition set according to the reagent and/or apparatus for preparing a smear (preparing condition ID) in the second example of the index setting process. As illustrated in FIG. 16, the controller 50 specifies, for example, from "Preparing condition ID of Reagent A/SP-10: C01", "Preparing condition ID of Reagent B/SP-10: C02," "Preparing condition ID of Reagent A/SP-50: C10" , and the like, a specific Preparing condition ID "C02" based on the user via the input part 90. Then, the controller 50 extracts one or more indexes associated with the preparing condition ID "C02" and control values associated with each of the indexes from the control index information MII stored in the storage 80, so as to set the indexes and the control values as the set index information SII. Note that the indexes included in the control index information MII are associated with the preparing condition ID based on the predetermined standard determined according to the smear preparing condition.

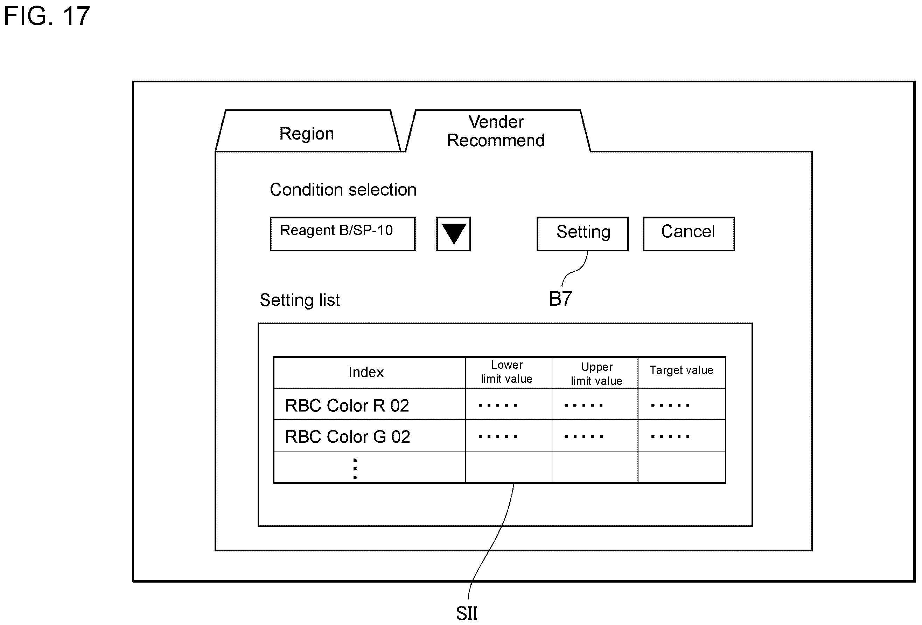

FIG. 17 is a diagram illustrating an example of a setting screen for condition selection in the second example of the index setting process. First, on the screen, multiple Vendor Recommend IDs, for example, "Reagent A/SP-10", "Reagent B/SP-10", "Reagent A/SP-50", etc. are displayed in a pull-down format for selection. As illustrated in FIG. 17, when the user selects one Vender Recommend ID (e.g., "Reagent B/SP-10") and presses a setting button B7, one or more indexes that are associated with Vender Recommend ID "Reagent B/SP-10" and control values that correspond to each of the indexes are extracted from the control index information MII, and set as the set index information SII.

In the setting screen, a condition(s) that is different from that in the Vender Recommend ID may be displayed being selectable, such that one or more indexes associated with a selected condition and control values associated with each of the selected one or more indexes may be extracted as the set index information SII. The "different condition" includes, for example, a preparing condition ID, or information on a staining solution, a pH of a buffer, or a staining time. Also, a plurality of selection columns may be displayed on the setting screen so that these plurality of conditions can be selected individually.

Note that a different control value(s) may be set for an index to be used for the quality control, even for the same type of index, depending on the Vendor Recommend ID or the preparing condition ID. For example, regarding the index "RBC Color R" in FIG. 16 and FIG. 17 the upper limit value, lower limit value, and target value set corresponding to "RBC Color R 01", "RBC Color R 02", "RBC Color R 03", etc., may differ from each other depending on the Vender Recommend ID or the preparing condition ID. Furthermore, each index may be set for a different type of index depending on the Vender Recommend ID or the preparing condition ID. For example, the types of indexes associated with the Vender Recommend ID "Reagent A/SP-10" (RBC Color S, RBC Redness Index, NEUT Granule Index, NEUT Cell Diameter) includes at least one different from the types of indexes associated with the Vendor Recommend ID "Reagent B/SP-10 (RBC RGB, RBC Redness Index, NEUT Nuclear Color G, and NEUT N/C). When the Region ID "Reagent B/SP-10" is specified, the indexes associated with "Reagent B/SP-10" and the control values associated with each of the indexes are extracted and set as the set index information SII.

### (Third example of index setting process)

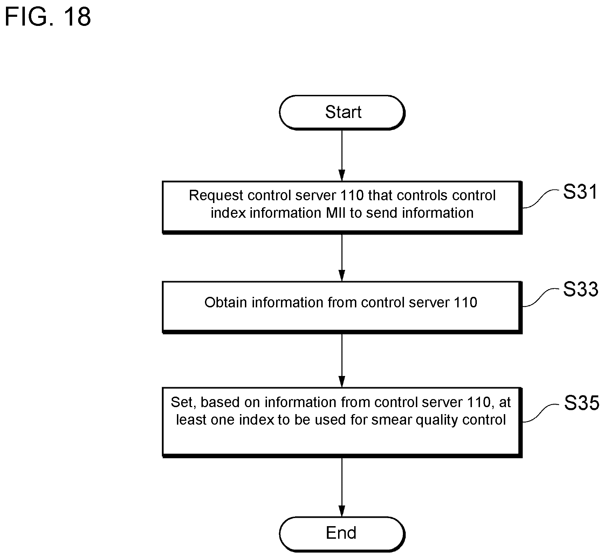

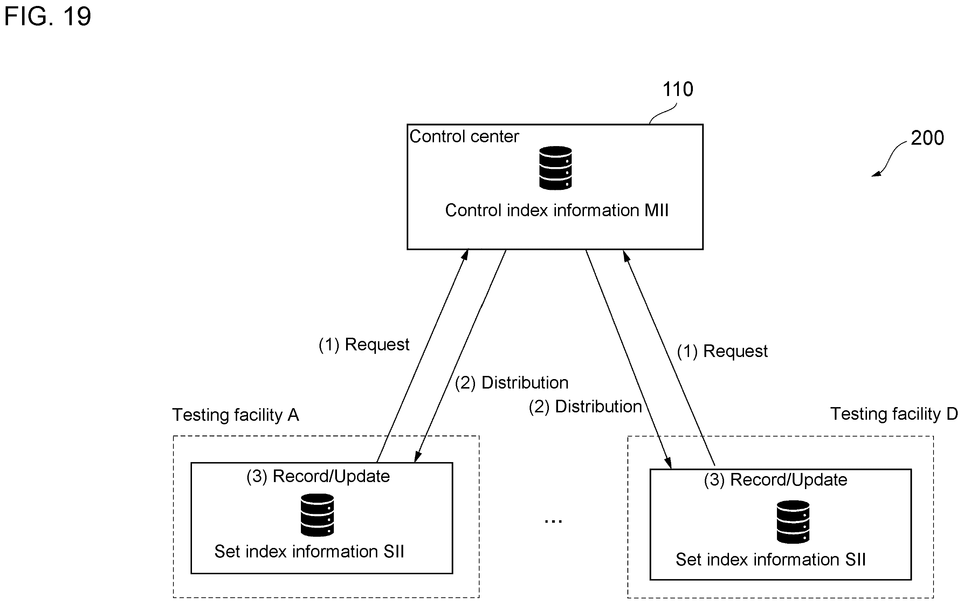

In a third example of an index setting process, based on index-related information that is transmitted from a control center (server) and includes information on an index(es) and a control value(s) to be used for the quality control, one or more testing systems set the index(es). FIG. 18 is a flowchart illustrating the third example of the index setting process. FIG. 19 is a diagram illustrating an example of information processing of the control center and the testing systems in the third example of the index setting process.

As illustrated in FIG. 18 and FIG. 19, the controller 50 of the quality control apparatus 1 requests the control center 110 that manages the control index information MII to send information (step S31 in FIG. 18/ "(1) Request" in FIG. 19). The control center 110 distributes the information to the controller 50. The controller 50 obtains the information from the control center 110 (step S33 in FIG. 18/ "(2) Distribution" illustrated in FIG. 19). The controller 50 sets or updates at least one index used for the smear quality control based on the information from the control center 110, and records the at least one index as the set index information SII (step S35 in FIG. 18 / "(3) Record/Update" illustrated in FIG. 19).

The information transmitted from the control center 110 in response to the request from the controller 50 of the quality control apparatus 1 may include, for example, at least a part of the control index information MII that includes indexes and control values according to a testing facility A, B, C, or D. Also, the information transmitted from the control center 110 may include information including the predetermined standard regarding the smear preparing condition. In addition, the control center 110 may automatically distribute, based on information of a testing facility A, B, C, or D, at least a part of the control index information MII that includes the indexes and the control values for the testing facility, regardless of whether the request is made or not. For example, the information of the testing facility A, B, C, or D may include information regarding a predetermined standard that is determined according to the type of the reagent and/or smear preparing apparatus used for preparing the smear or a predetermined standard for the smear preparing condition adopted in the testing facility.

The control center 110 is a facility provided with one or more servers or information processors. The control center 110 may be, for example, a facility of a vendor that provides analysis or quality control software, etc., or may be a central facility of a group of facilities in which multiple testing facilities collaborate. The control center 110 may be a cloud system that can be connected to any of the above facilities via a predetermined communication network.

Note that in the example of FIG. 19, in a control system 200, one control center 110 is provided, but the number of control centers is not limited thereto and is arbitrary. The number of testing facilities A to D in the control system 200 is also arbitrary.

### (Process of obtaining feature value)

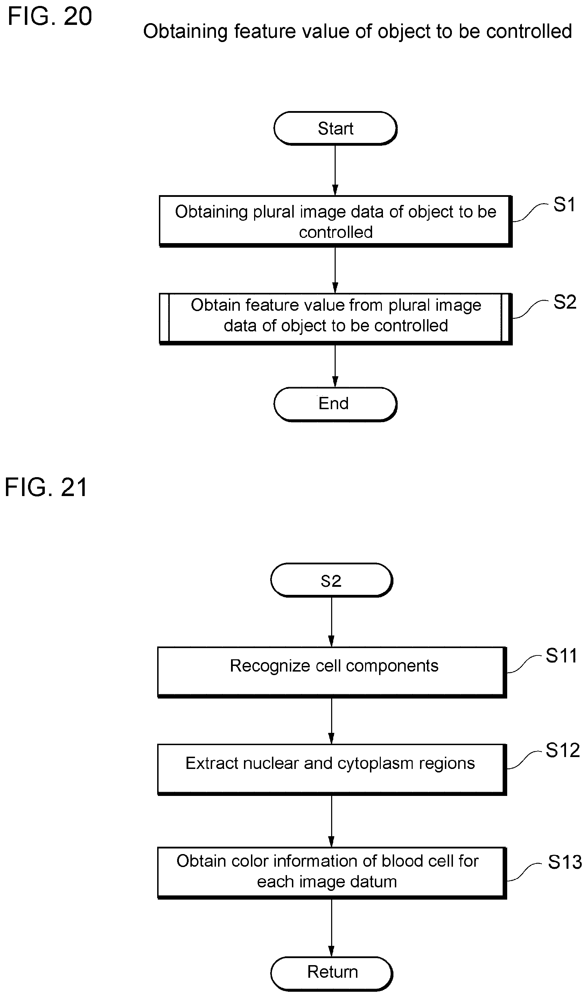

FIG. 20 is a flowchart illustrating a process of obtaining a feature value that reflects a staining state of a smear which is an object to be controlled.

As illustrated in FIG. 20, the controller 50 of the quality control apparatus 1 illustrated in FIG. 2 obtains a plurality of image data of the object to be controlled (step S1). The controller 50 obtains a feature value from the plurality of image data of the object to be controlled (step S2), the controller 50 obtains a feature value that reflects a staining state of the smear, which is the object to be controlled, by executing steps S1 to S2.

FIG. 21 is a flowchart illustrating the feature obtaining process of step S2 in FIG. 20. As illustrated in FIG. 21, the controller 50 illustrated in FIG. 5 recognizes each cell component based on the obtained plural image data IDs (step S11). Next, the controller 50 identifies and extracts regions of each cell including nucleus and cytoplasm based on the recognition result in step S11 (step S12). The controller 50 obtains color information of the blood cell in each of the extracted regions (feature value reflecting the staining state of the smear) for each of the plural image data IDs (step S13).

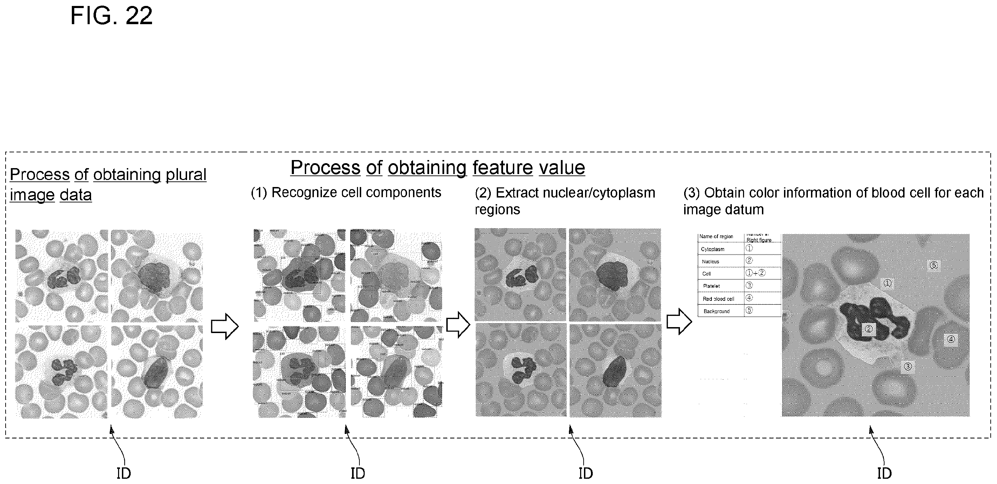

FIG. 22 is a diagram illustrating details of a process of obtaining image data of a smear to be controlled and a process of obtaining a feature value. As illustrated in FIG. 22, the controller 50 illustrated in FIG. 2 obtains, for example, hundreds or thousands of image data IDs from each of a plurality of smears. More specifically, the controller 50 obtains the plural image data IDs that are obtained by capturing by the smear image capturing apparatus 40 of the testing system 70-1 to 70-4.

The controller 50 obtains a feature value that reflects the staining state of the smear from each of the plurality of image data IDs. As illustrated in FIG. 22, the controller 50 executes (1) recognition of cellular components, (2) extraction of nuclear and cytoplasm regions, and (3) obtaining color information of the blood cell for each image data, as the feature value obtaining process.

More specifically, the controller 50 recognizes each cell component based on the obtained plural image data IDs as a process (1). Next, the controller 50 identifies and extracts regions of each cell including, for example, the nucleus and cytoplasm based on the recognition result of the process (1), as a process (2). As a process (3), for each of the plural image data IDs, the controller 50 obtains color information of the blood cell (feature value reflecting the staining state of the smear) with associating information on cell types (red blood cell, white blood cell, platelet, etc.) and information on structural components (nucleus, cytoplasm, granule, etc.) in each extracted region.

The feature value that reflects the staining state of the smear includes the color information obtained from the regions of the blood cell in the image data, as described above. The "feature value that reflects the staining state of the smear" includes, for example, at least one of the values of luminance, hue, saturation, and brightness values of the color components (e.g., Red, Green, Blue) obtained from the image data of the blood cell, and their combined values (e.g., HSV value, RGB).

The "blood cell" includes, for example, at least one of the red blood cell, white blood cell, and platelet. The white blood cell includes, for example, at least one of basophil, eosinophil, neutrophil, monocyte, and lymphocyte.

The red blood cells, which account for about 90% of the blood cells in blood, also accounts for a large proportion (area) of the smear. Therefore, the color information of the red blood cells is more preferable as the feature value that reflects the staining state of the smear, because the color information of the red blood cells can be information that reflects the staining state of the entire smear. The color information of the red blood cells tends to reflect a change in the staining state due to a difference in the staining condition, such as pH, etc., more easily than the color information of the white blood cells. On the other hand, the color information of the white blood cells may be adopted as the feature value that reflects the staining state of the smear. In this case, multiple types of feature values that reflect the staining state of the smear, such as the red blood cell, white blood cell, etc., of the smear may be used for the quality control. For example, the quality of the staining state of the entire smear can be managed by using of at least one component of the color information of the red blood cells and at least one component of the color information of the white blood cells, while the stainability of components (e.g., nuclei or granules) of the white blood cells, which are important objects of analysis, can also be managed.

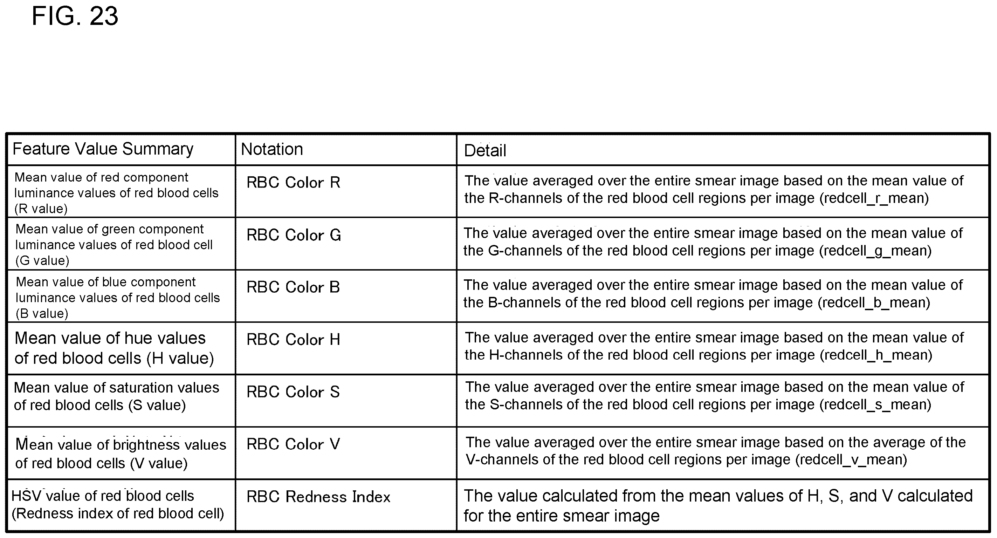

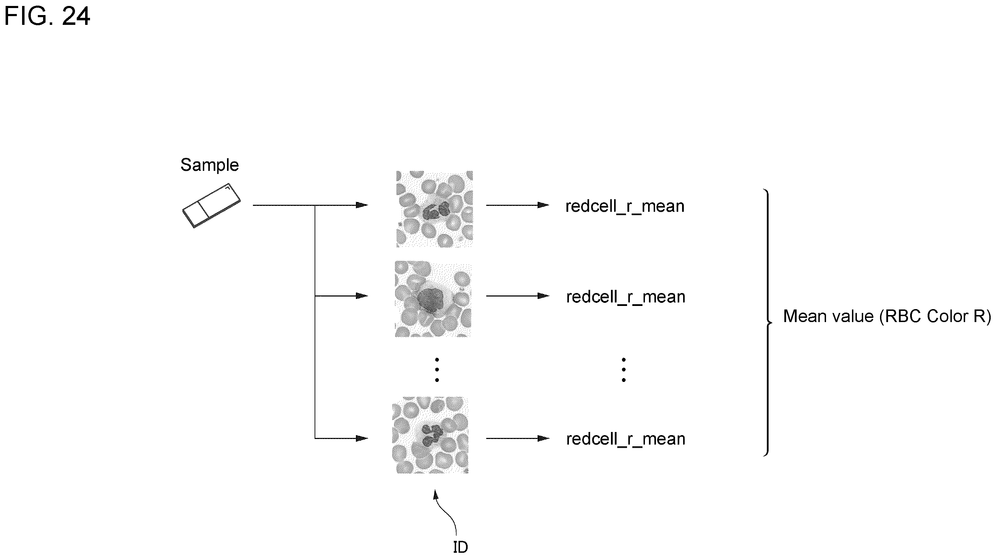

FIG. 23 is a diagram illustrating an example of feature values that reflect a staining state of red blood cells. As illustrated in FIG. 23, the feature values that reflects the staining state of the red blood cells includes, for example, a mean value of luminance values of color components of the red blood cells, a mean value of hue values of the red blood cells, a mean value of saturation values of the red blood cells, a mean value of brightness values of the red blood cells, and an HSV value of the red blood cells. Here, with reference to FIG. 24, an example of a process of obtaining the mean value of the luminance values of the red components of the red blood cells, as an example of one of the feature values, is described.

As illustrated in FIG. 24, the controller 50 illustrated in FIG. 2 obtains a plurality of image data IDs generated by imaging one smear (sample). Next, the controller 50 obtains a mean value of the red (R) components of the red blood cell regions (redcell_r_mean) for each of the plurality of obtained image data IDs. Then, the controller 50 obtains, based on all the plurality of image data IDs (number of images) of the one smear, a mean value (RBC Color R) of the mean values of the red (R) components of the red blood cell regions (redcell_r_mean) for the plurality of the obtained image data IDs.

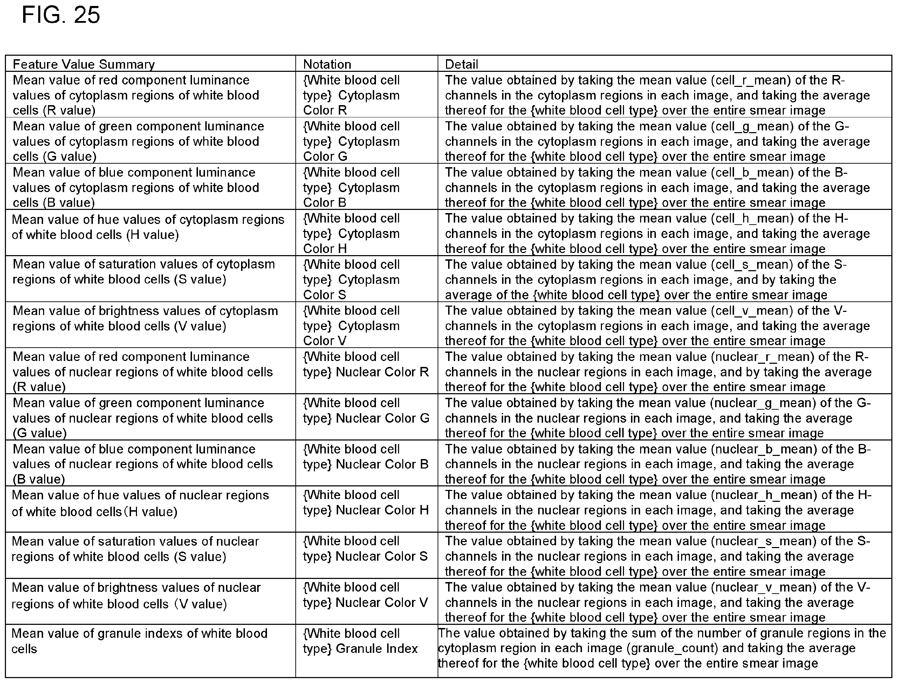

The feature values obtained from the image data includes feature values corresponding to the color information that are obtained from the structural components, such as nucleus, cytoplasm, granule, and the like, of the white blood cells. These feature values reflect the stainability of the structural components of the white blood cells. A specific example of the feature values obtained from the white blood cells is described with reference to FIG. 25.

FIG. 25 is a diagram illustrating an example of the feature values that reflect the stainability of the white blood cells. As illustrated in FIG. 25, the feature values that reflect the stainability of the white blood cells includes, for example, a mean value of luminance values in each color component of a cytoplasm regions of the white blood cells, a mean value of hue values of the cytoplasm regions of the white blood cells, a mean value of saturation values of the cytoplasm regions of the white blood cells, and a mean value of brightness values of the cytoplasm regions of the white blood cells. The feature values that reflect the stainability of the white blood cells include, for example, a mean value of luminance values in each color component of nuclear regions of the white blood cells, a mean value of hue values of the nuclear regions of the white blood cells, a mean value of saturation values of the nuclear regions of the white blood cells, and a mean value of brightness values of the nuclear regions of the white blood cells. Also, the feature values that reflect the stainability of the white blood cells may include values of standard deviations with respect to the luminance values, hue values, saturation values, and brightness values in the cytoplasm regions and the nuclear regions of the white blood cells. The feature values that reflect the stainability of the white blood cells includes, for example, a mean value of granule indexes of the white blood cells. The feature values includes feature values that reflect forms of the blood cells.

The feature values that are obtained from the blood cells in the smear image data include feature values that reflect the information of the forms of the blood cells, in addition to the "feature values that reflect the staining state of the smear" described above.

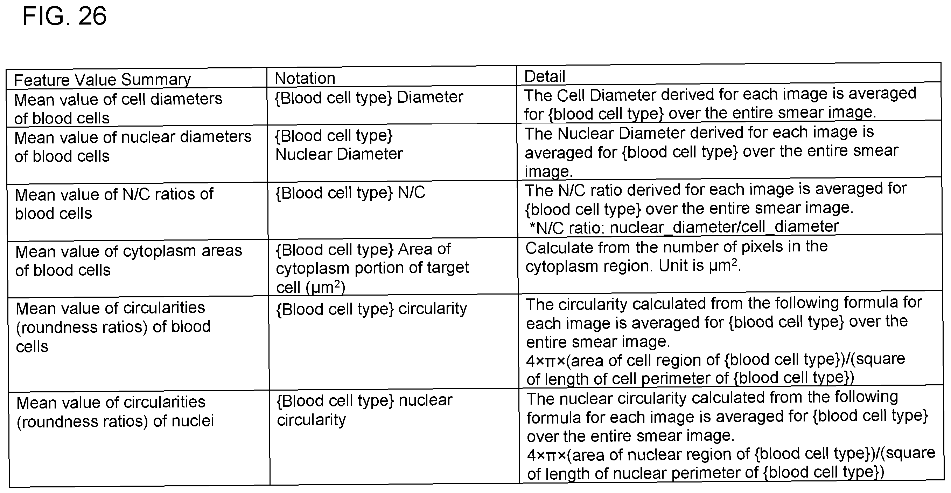

FIG. 26 is a diagram illustrating an example of feature values that reflect forms of blood cells. As illustrated in FIG. 26, the feature values that reflect the blood cell forms obtained from the blood cells includes, for example, a mean value of cell diameters of the blood cells, a mean value of nuclear diameters of the blood cells, a mean value of N/C ratios of the blood cells, a mean value of areas of cytoplasm of the blood cells, a mean value of circularity (roundness ratios) of the blood cells, and a mean value of circularity of nuclei of the blood cells.

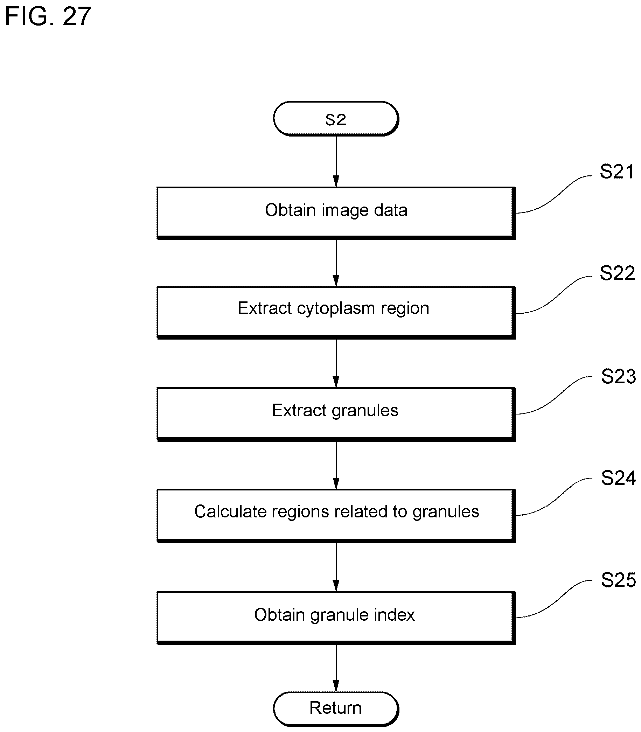

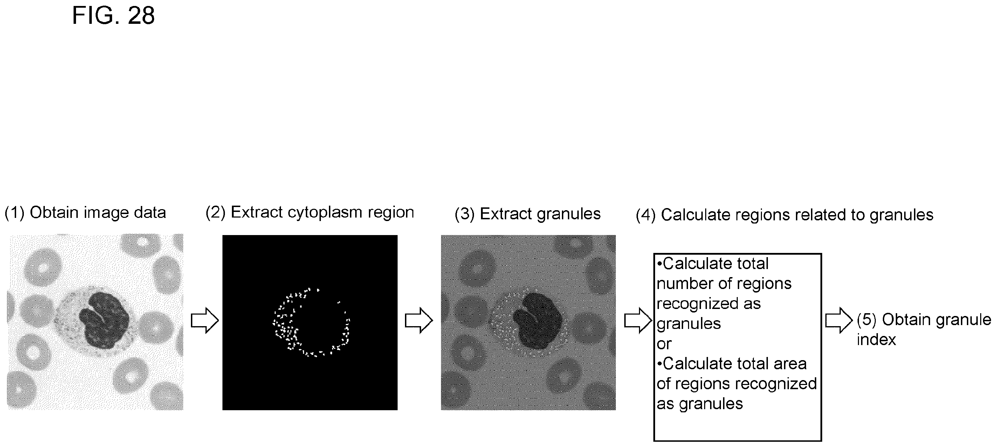

Here, referring to FIG. 27 and FIG. 28, an example of a process of obtaining a granule index (Granule_Index), as an example of a feature value, of neutrophils among white blood cells is described.

FIG. 27 is a flowchart illustrating an example of a process of obtaining a granule index, which corresponds to the process of obtaining the feature value in step S2 of FIG. 20. As illustrated in FIG. 27, the controller 50 illustrated in FIG. 2 obtains image data, for example, including the white blood cells, in the smear (step S21). The controller 50 executes, for example, a local binarization process on the obtained image data to extract cytoplasm regions (step S22). The controller 50 extracts granules (step S23). Specifically, the controller 50 identifies granules (e.g., granules each of which includes a few pixels or more) based on a predetermined number of pixels from the extracted cytoplasm regions. The controller 50 obtains a granule size (area) and an average luminance for each of the identified granules. The controller 50 classifies the identified granules into primary granules and secondary granules by determining predetermined threshold values for each of the granule size and the difference between the granule average luminance and the cytoplasm average luminance.

Next, the controller 50 calculates regions related to the granules (step S24). Specifically, the controller 50 calculates the total number of the primary granules and secondary granules in the granule regions recognized as granules. The controller 50 may calculate the total area of the regions recognized as granules (total granule area) or the total granule area ratio of the regions recognized as granules. The controller 50 obtains at least one of the calculated total number of granules, the calculated total granule area, and the calculated total granule area ratio of the granule regions as a granule index (step S25).

Here, referring to FIG. 28, an example of a process of obtaining a granule index (Granule_Index) relating to, for example, neutrophils among white blood cells as an example of a feature value is described. As illustrated in FIG. 28, (1) the controller 50 illustrated in FIG. 2 obtains image data including the white blood cells, in a smear. (2) The controller 50 extracts cytoplasm regions by executing, for example, a local binarization process on the obtained image data. Here, the "granule index" is a feature value that reflects the number of granules included in the white blood cells, and can be obtained based on the number or area of granule regions identified in the cytoplasm regions of the white blood cells. The "granule region" can be identified based on the binarized image data to distinguish from the cytoplasm region. By obtaining the granule index, a change in granules in blood cells can be objectively and quantitatively indicated, which may be used for the diagnosis and evaluation of a disease with an increase or decrease in granules, such as an infectious disease, in which granules increase, myelodysplastic syndromes (MDS: myelodysplastic syndromes), in which granules decreases, etc.

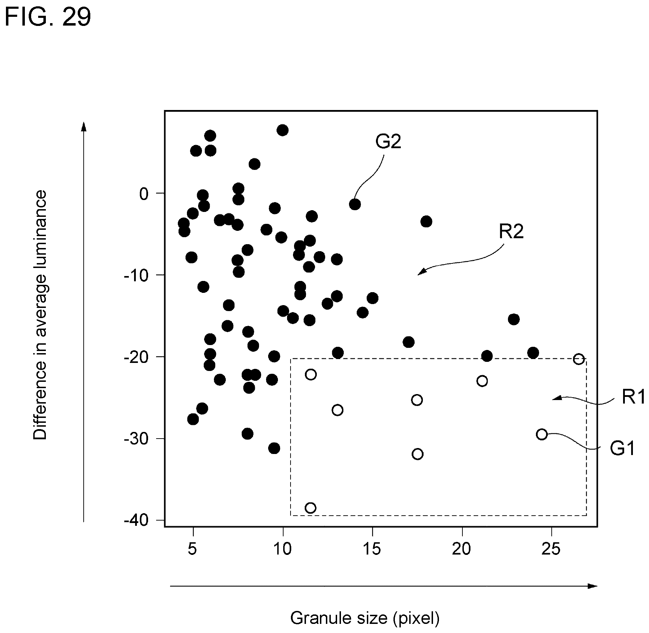

(3) The controller 50 identifies granules (e.g., granules each of which includes a few pixels or more) based on a predetermined number of pixels from the extracted cytoplasm region. The controller 50 obtains a granule size (area) and an average luminance for each identified granule. The controller 50 classifies the identified granules into the primary granules and the secondary granules by determining predetermined threshold values for each of the granule size and the difference between the granule average luminance and the cytoplasm average luminance (e.g., a difference between an average luminance of a portion of the cytoplasm other than the granule portions and the average luminance of the granule portions). Here, when the difference between the average luminance of the granule and the average luminance of the cytoplasm is 0 (zero), it indicates that the luminance is the same as that of the background portion of the image data. Also, when the difference between the average luminance of the granule and the average luminance of the cytoplasm is large in the negative direction, it indicates that the granule area is thicker (darker) than the other areas. A process of classifying the granule is described in more detail with reference to FIG. 29.

FIG. 29 is a diagram illustrating an example of a process of extracting primary granules and secondary granules. For example, when the threshold value of the granule size of the secondary granules is set to "10" pixels and the threshold value of the difference between the average luminance of the granule and the average luminance of the cytoplasm is set to "0", the controller 50 classifies the identified granules into primary granules G1, which are included in a region R1 surrounded by a dashed line in the graph in FIG. 29, and secondary granules G2, which are included in a region R2 different from the region R1 in the graph in FIG. 29. Each of the above threshold values can be arbitrarily set based on the resolution of the image data, for example, and can be changed as appropriate. Also, the size of one pixel may be set as desired, for example, approximately 0.01 µm² .

Returning to FIG. 28, (4) the controller 50 calculates the total number of the primary granules and the secondary granules in the granule regions recognized as granules. The controller 50 may calculate the total area of the regions recognized as the granule (total granule area) or the total granule area ratio of the regions recognized as the granule. Here, since there is a certain correlation between the total number of granules in the granule region, and the total granule area and the total granule area ratio, the total granule area and the total granule area ratio can be adopted as feature values in addition to or instead of the total number of granules in the granule regions. The total granule area ratio is, for example, a ratio of how much of the cytoplasm area is occupied by the granule area. (5) The controller 50 obtains at least one of the calculated number of granules in the granule regions, and the calculated total granule area and the calculated total granule area ratio as the granule index.

### (Example of output screen for quality control information)

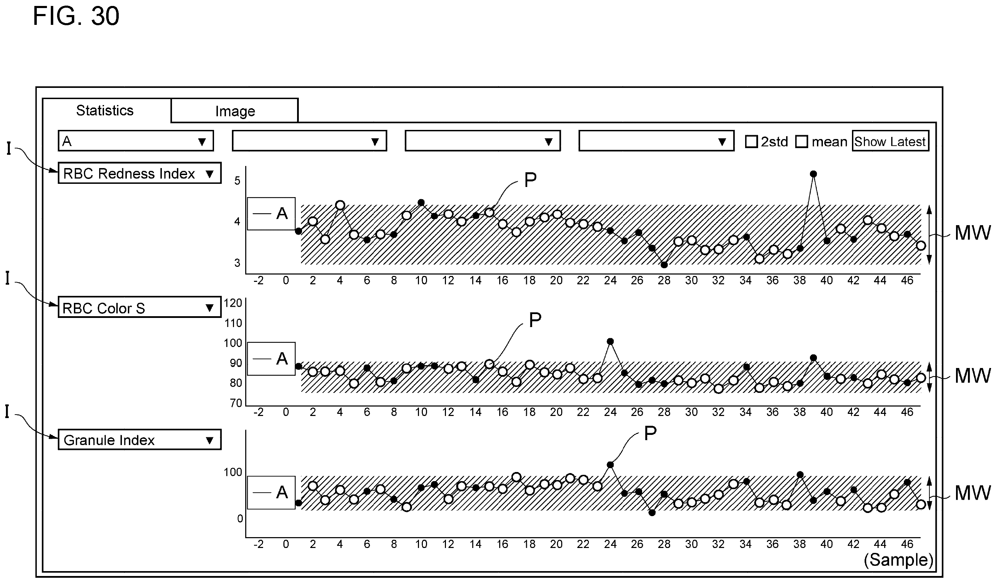

FIG. 30 is a diagram illustrating an example of an output screen of quality control information and feature values reflecting staining states of smears to be controlled. FIG. 30 is an example of the output screen for internal quality control outputting the quality control information and the feature values reflecting the staining states of the smears to be controlled. The quality control apparatus 1 illustrated in FIG. 1 obtains a plurality of feature values from each of the smears prepared in a single testing facility and outputs the quality control information determined based on those feature values. As illustrated in FIG. 30, the quality control apparatus 1 outputs, for each index I (e.g., RBC Redness Index, RBC Color S, Granule Index), plots P of the plurality of feature values that are related to the color information of the red blood cells and plots P of the plurality of feature values that are related to the color information of the white blood cells along with a control width MW. According to the configuration, it is possible to understand the staining state of the entire smear based on the color information of the red blood cells, and to perform the quality control for the stainability and the form of the white blood cells, which is important analysis targets, as well.

The RBC Redness Index, which is the index I of the feature value, is a value that is calculated from the mean values of the hue values of the red blood cells, the saturation values of the red blood cells, and the brightness values of the red blood cells by conducting principal component analysis, respectively. RBC Color S, which is the index I of the feature value, is the saturation value of the red blood cells. Granule Index, which is the index I of the feature value, is the granule index of the white blood cells.

The quality control apparatus 1 may output only plots P of a plurality of feature values related to the color information of the red blood cells to the output screen, or may output only plots P of a plurality of feature values related to the color information of the white blood cells to the output screen.

The quality control information for the internal quality control is information that indicates variation in the staining states of the smears within a predetermined single testing facility. The quality control information can serve as a standard for the quality control according to the staining condition of the predetermined single testing facility. Therefore, the quality of the smears prepared daily in the testing facility can be controlled based on the standard appropriate for the testing facility.

The quality control information for the internal quality control can also be used in the following situations. For example, the quality control information for the internal quality control is used to check whether there is any problem with the staining state(s) of a smear(s) obtained after maintenance of the testing apparatus, after changing a reagent, or at a change of a reagent lot. Also, according to the quality control information for the internal quality control, an abnormality of the reagent or the testing apparatus can be identified in advance by checking the trend of rise or fall of the feature value over time. Furthermore, since an environmental condition, such as temperature, humidity, etc., affects the staining state of a smear, the quality control information for the internal quality control can also be used to optimize the staining condition in response to a change in the environmental condition.

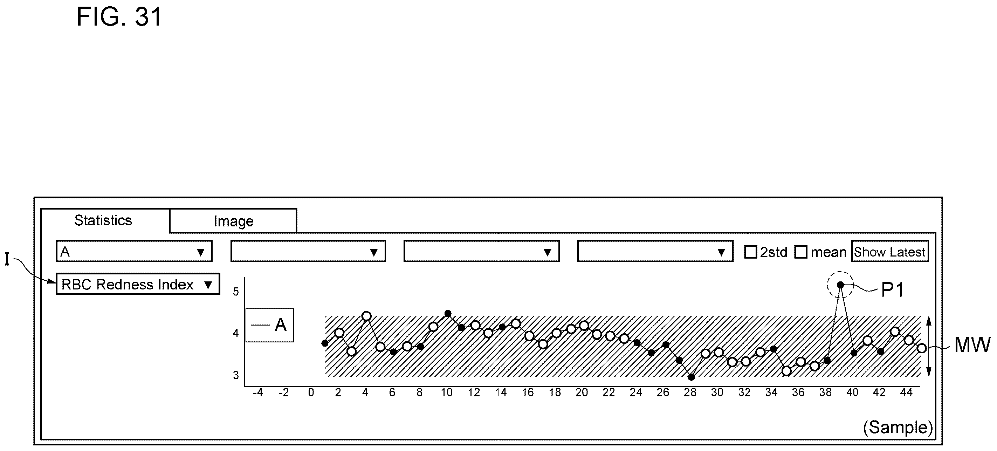

FIG. 31 is a diagram illustrating an example of an output screen of quality control information and feature values that reflect staining states of smears to be controlled. The quality control information and the feature values that reflect the staining states of the smears to be controlled are output in such a way that it is possible to identify whether any one of the feature values reflecting the staining states of the smears to be controlled is within a predetermined range. In the example of the screen illustrated in FIG. 31, as a plot P1 having a specific feature value is out of the control width MW, the plot P1 having the specific feature value is output in a form different from the other plots on the output screen. For example, the plot P1 is output with more emphasis than the other plots. More specifically, the plot P1 is output in a different color, a different size, or a different shape than the other plots (e.g., the plot P1 is in a round shape and the other plots are in square shapes.). According to the configuration, it is possible to easily distinguish a plot(s) having a feature value(s) that is not included within a predetermined range from other plots that are included within the predetermined range.

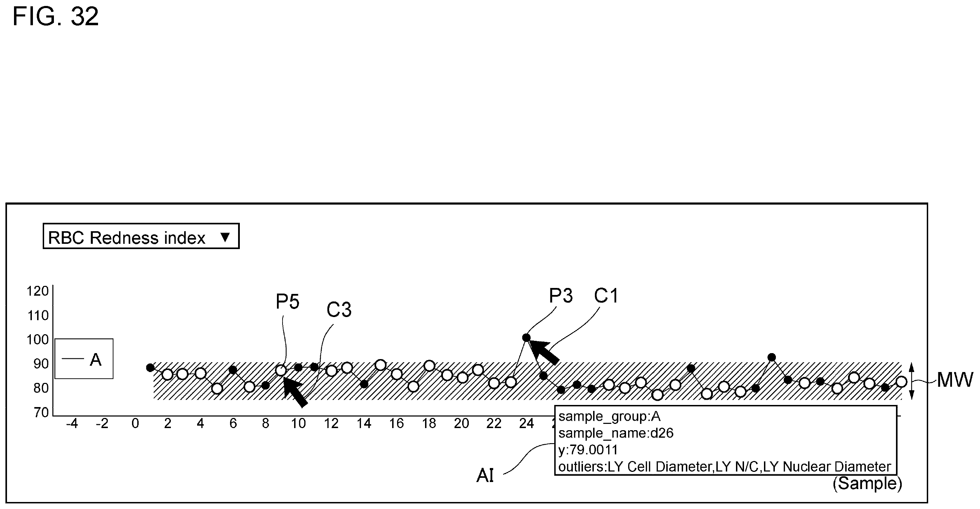

FIG. 32 is a diagram illustrating an example of an output screen including a predetermined alert indication for a plot having a specific feature value that is out of a control width MW. As illustrated in FIG. 32, when a first specific operation is executed by the user for a plot P3 having a specific feature value that is out of the control width MW, an alert indication AI including detailed information on the specific feature value of the plot P3 is output. The first specific operation by the user is arbitrary, but for example, includes an operation of stopping a cursor C1 which corresponds to a mouse movement operated by the user, on the plot P3 for a certain period of time.

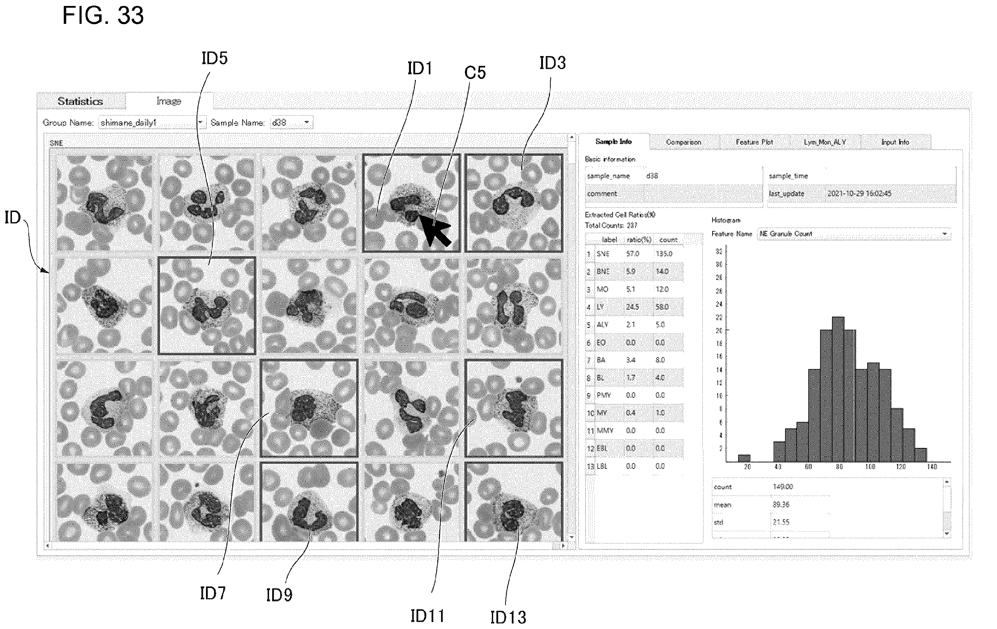

According to the configuration, detailed information concerning the specific feature value that is out of the control width can be appropriately presented to the user. Therefore, the user can easily identify the cause of the feature value being out of the control width by seeing the detailed information. When a one-click operation is performed with the mouse when the cursor C1 is stopped on the plot P3, a screen that displays a plurality of image data associated with the feature value of the plot P3 may be displayed, such as being illustrated in FIG. 33 below.

FIG. 33 is a diagram illustrating an example of a screen that displays a plurality of image data obtained from a single smear. In particular, FIG. 33 illustrates a screen that is output, for example, when the user performs a predetermined operation on the screen illustrated in FIG. 32. For example, when a second specific operation is performed by the user on a plot P5 having a specific feature value on the output screen illustrated in FIG. 32, the screen illustrated in FIG. 33 is output, which displays a plurality of image data IDs that are used in obtaining the specific feature value of the plot P5.

According to the configuration, the user can easily check a list of the plurality of image data IDs that are used in obtaining the specific feature value as desired by the user. The second specific operation by the user is arbitrary and, for example, includes a double-click operation with the mouse when the cursor C3 is stopped on the plot P5. Also, it is preferable that the first specific operation and the second specific operation by the user are different operations, but they may be the same operation.

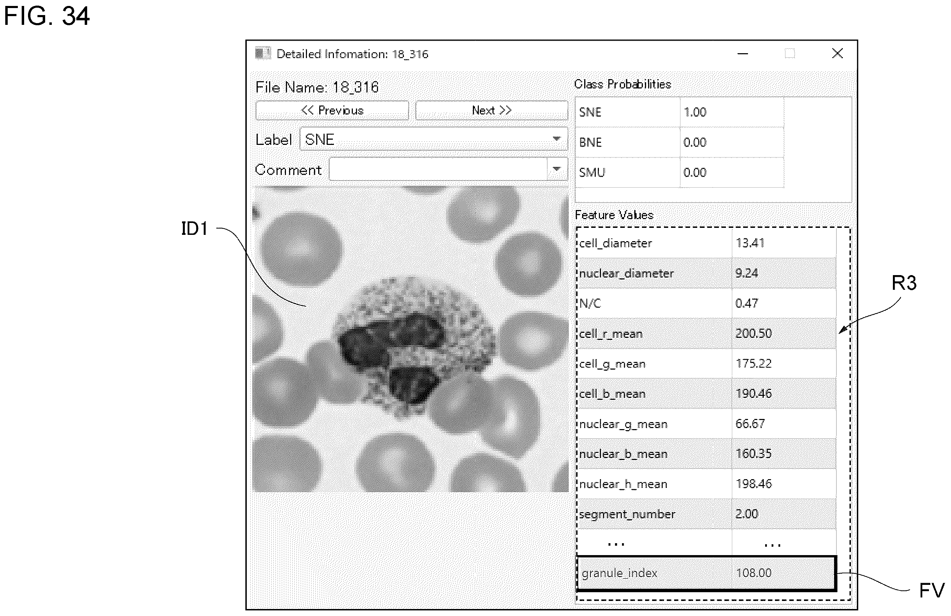

FIG. 34 is a diagram illustrating an example of a screen displaying specific image data in association with a plurality of feature values (Feature Values) that are associated with the specific image data. In particular, FIG. 34 is a screen that is output when the user performs a predetermined operation on the screen illustrated in FIG. 33, for example. For example, when a third specific operation is performed by the user for a specific image data ID1 on the output screen illustrated in FIG. 33, the screen illustrated in FIG. 33 is output which includes an area R3 surrounded by a dashed line and displaying detailed information on a plurality of feature values corresponding to the selected specific image data ID1.

According to the configuration, the user easily check a list of the plurality of feature values corresponding to the specific image data desired by the user. Note that the third specific operation by the user is arbitrary, and, for example, includes a double-click operation with the mouse when a cursor C5 is stopped on the image data ID1.

Here, a feature value FV (e.g., granule_Index) that is out of a preset normal range may be displayed in a different form from other feature values. According to the configuration, the specific feature value FV that is an abnormal value can be easily distinguished from other (normal) feature values. The normal range may be set based on a control value for an index of the quality control (e.g., information on the control width obtained by the upper limit value and the lower limit value).

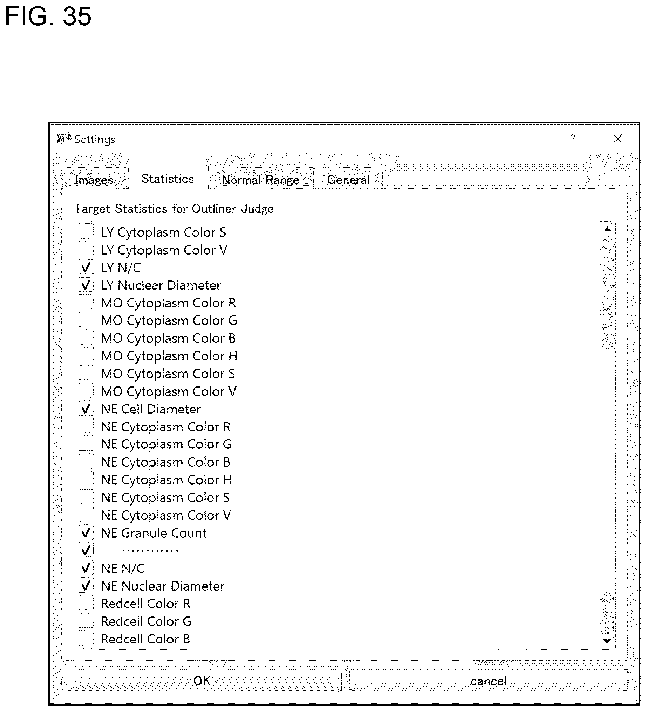

FIG. 35 is a diagram illustrating an example of a screen on which a feature value(s) to be obtained can be selected. The feature value(s) to be obtained or the feature value(s) that are to be output on the screens illustrated in FIG. 30 to FIG. 32 are predetermined for each testing facility or region, etc., for example. On the other hand, it is possible for the user to select one or more feature values desired by the user by operating a feature value selection screen such as being illustrated in FIG. 35. According to the configuration, the feature value(s) desired by the user can be output on an output screen for the feature value(s) that reflects the staining state of the smear to be controlled and the quality control information.

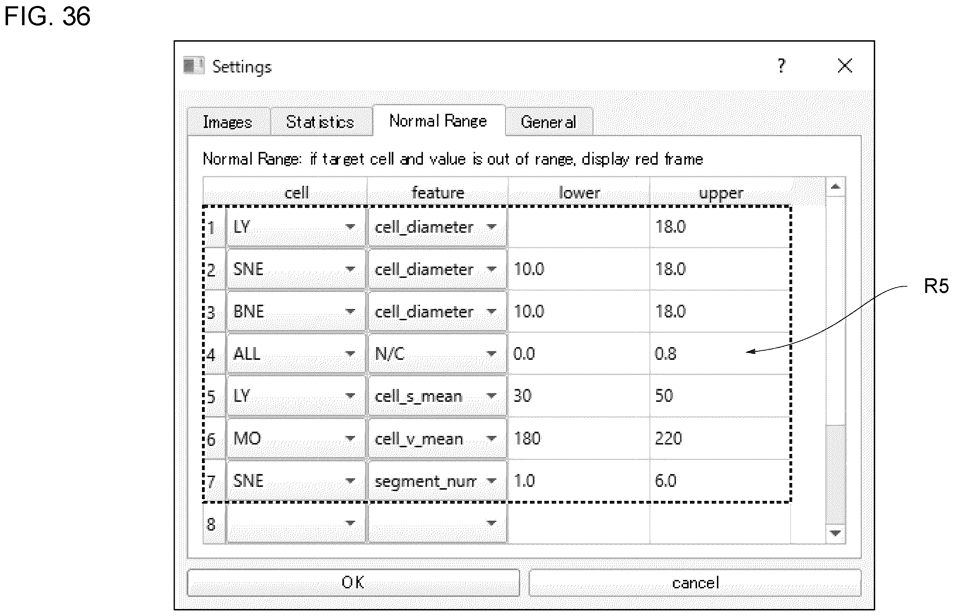

FIG. 36 is a diagram illustrating an example of a screen on which a normal range (control width) can be set for any one of feature values. In the screen illustrated in FIG. 36, the user can set, for example, based on a result of quality control conducted at each testing facility, a control width for any feature value in a region R5 surrounded by a dashed line. For example, each of a set of values (lower, upper) in the fifth row (cell column is "LY", and feature column is "cell_s_mean") indicates a control width of the feature value related to the staining state of cytoplasm of a lymphocyte. Each value (lower, upper) of an item in the sixth row (cell column is "MO", and feature column is "cell_v_mean") indicates a control width of the feature value related to the staining state of cytoplasm of a single cell. In addition, each value of an item in the seventh row (cell column is "SNE", and feature column is "segment_num") indicates a control width of the feature value related to the number of lobulated segments of a neutrophil. According to the configuration, the user can optionally set the control width for the feature value based on an index(es) of the quality control at a testing facility, thus it is possible to suppress a subjective judgment error between inspectors.

The image data that has an abnormal feature value, which is out of the control width illustrated in FIG. 36 may be displayed in a form different from other image data IDs that have normal feature values, such as image data ID1, ID3, ID5, ID7, ID9, ID11, ID13 among the plural image data displayed on the screen illustrated in FIG. 33. A method of displaying the image data in such a different form is arbitrary, but may include, for example, putting a colored frame only to the image data that has the abnormal feature value or displaying the image data that has the abnormal feature value larger than the other image data that have the normal feature values on the screen.

### (Other embodiments)

The above embodiments are intended to facilitate understanding of the invention and are not to be construed as limiting the invention. The invention may be changed/improved (e.g., combining the embodiments, omitting some configurations of each embodiment) without departing from its intent, and the invention also includes equivalents thereof.

For example, although the quality control information of a smear (a smear specimen) of blood collected from a subject is output, the quality control information of a cell specimen collected from a subject may also be output.

For example, although the smear slide 10 is prepared by the smear preparing apparatus 20 included in the quality control system 100, the smear slide 10 may be manually prepared by a laboratory technician belonging to a testing facility. Also, the image(s) of the smear slide 10 is captured by the smear image capturing apparatus 40; however, may be manually captured by a laboratory technician belonging to a testing facility.

One or more embodiments described above may be summarized as follows.

A method of quality control for a specimen that is prepared from a sample according to a first aspect includes: (S1) setting, from a control index information (MII) in which a plurality of indexes for the specimen are respectively associated with control values corresponding to the plurality of indexes, at least one index to be used for the quality control for the specimen; (S3) obtaining, from image data of the specimen, a feature value relating to the at least one index; and (S5) outputting, based on the feature value and the control value that is associated with the at least one index, quality control information for the specimen.

In the method of quality control according to the first aspect, the quality control information may be a chart or notification information displaying the feature value and the control value in a comparable manner.

In the method of quality control according to the first aspect, the index may be set according to a selection from the control index information.

In the method of quality control according to the first aspect, the index may be set based on a predetermined standard for the specimen quality control.

In the method of quality control according to the first aspect, the index may be set based on a guideline for the specimen quality control.

In the method of quality control according to the first aspect, the index may be set based on the guideline that is determined according to a region in which the specimen is prepared.

In the method of quality control according to the first aspect, the index may be set based on a predetermined standard regarding a specimen preparing condition.

In the method of quality control according to the first aspect, the index may be set based on the predetermined standard that is determined according to a type of a reagent used for preparing the specimen and/or a type of a specimen preparing apparatus.

In the method of quality control according to the first aspect, the index may be set based on information transmitted from a server that controls the control index information.

In the method of quality control according to the first aspect, the information may include a predetermined standard regarding a specimen preparing condition.

In the method of quality control according to the first aspect, the specimen is prepared from the sample that includes cells collected from a subject.

In the method of quality control according to the first aspect, the specimen may be a blood smear that is prepared from a blood collected from a subject.

In the method of quality control according to the first aspect, the index may include at least color information of cells in the image data of the specimen.

In the method of quality control according to the first aspect, the cells may be blood cells.

A quality control apparatus to perform quality control of a specimen that is prepared from a sample according to a second aspect includes a controller (50), wherein the controller (50) is configured to: (S1) set, from control index information (MII) in which a plurality of indexes for the specimen are respectively associated with control values corresponding to the plurality of indexes, at least one index to be used for the quality control of the specimen; (S3) obtain, from image data of the specimen, a feature value relating to the at least one index; and (S5) output, based on the feature value and the control value that is associated with the at least one index, quality control information for the specimen.

A program according to a third aspect is a program that causes a quality control apparatus that performs quality control of a specimen prepared from a sample to perform operations including: (S1) setting, from control index information (Mll) in which a plurality of indexes for the specimen are respectively associated with control values corresponding to the plurality of indexes, at least one index to be used for the quality control of the specimen; (S3) obtaining, from image data of the specimen, a feature value relating to the at least one index; and (S5) outputting, based on the feature value and the control value that is associated with the at least one index, quality control information for the specimen.

### (Description of the sign)

1: Quality control apparatus, 10: Smear slide, 20: Smear preparing apparatus, 30: Smear transporting apparatus 40: Smear image capturing apparatus, 50: Controller, 60: Display part, 70-1 to 70-4: Testing system, 77: Communication part, 80: Storage, 90: Input part, 100: Quality control system, 110: Control center, 200: Control system
Further embodiments are defined as E1 to E15, as follows:
E1. A method of quality control for a specimen that is prepared from a sample comprising:
   setting, from a control index information in which a plurality of indexes for the specimen are respectively associated with control values corresponding to the plurality of indexes, at least one index to be used for the quality control for the specimen;
   obtaining, from image data of the specimen, a feature value relating to the at least one index; and
   outputting, based on the feature value and the control value that is associated with the at least one index, quality control information for the specimen.
E2. The method of quality control according to embodiment 1, wherein
   the quality control information is a chart displaying the feature value and the control value in a comparable manner or notification information displaying the feature value and the control value in a comparable manner.
E3. The method of quality control according to embodiment 1 or 2, wherein
   the index is set according to a selection from the control index information.
E4. The method of quality control according to any one of embodiments 1 to 3, wherein
   the index is set based on a predetermined standard for the specimen quality control.
E5. The method of quality control according to any one of embodiments 1 to 4, wherein
   the index is set based on a guideline for the specimen quality control.
E6. The method of quality control according to embodiment 5, wherein
   the index is set based on the guideline that is determined according to a region in which the specimen is prepared.
E7. The method of quality control according to any one of embodiments 1 to 4, wherein
   the index is set based on a predetermined standard regarding a specimen preparing condition.
E8. The method of quality control according to embodiment 7, wherein
   the index is set based on the predetermined standard that is determined according to a type of a reagent used for preparing the specimen and/or a type of a specimen preparing apparatus.
E9. The method of quality control according to any one of embodiments 1 to 8, wherein
   the index is set based on information transmitted from a server that controls the control index information.
E10. The method of quality control according to embodiment 9, wherein
   the information includes a predetermined standard regarding a specimen preparing condition.
E11. The method of quality control according to any one of embodiments 1 to 10, wherein
   the specimen is prepared from the sample that includes cells collected from a subject.
E12. The method of quality control according to embodiment 11, wherein
   the specimen is a blood smear that is prepared from a blood collected from a subject.
E13. The method of quality control according to any one of embodiments 1 to 12, wherein
   the index includes at least color information of cells in the image data of the specimen.
E14. The method of quality control according to embodiment 13, wherein
   the cells are blood cells.
E15. A quality control apparatus to perform quality control of a specimen that is prepared from a sample, comprising a controller, wherein
   the controller is configured to:
   set, from control index information in which a plurality of indexes for the specimen are respectively associated with control values corresponding to the plurality of indexes, at least one index to be used for the quality control of the specimen;
   obtain, from image data of the specimen, a feature value relating to the at least one index; and
   output, based on the feature value and the control value that is associated with the at least one index, quality control information for the specimen.

## Claims

1. A system comprising:
a specimen preparing apparatus configured to prepare a specimen by smearing a sample on a slide and staining the sample on the slide;
an image capturing apparatus configured to capture an image of the specimen;
a specimen transporting apparatus configured to transport the specimen to the image capturing apparatus; and
a computer communicably connected to the image capturing apparatus, wherein the computer comprises a controller configured to perform a quality control of the specimen preparing apparatus that operates according to a predetermined condition for preparing the specimen, the controller being configured to:
obtain a feature value of the image based on a color of components of the specimen in the image, wherein the feature value is quantified information that reflects a staining condition of the specimen; and
output quality control information based on the feature value and a predetermined standard corresponding to the predetermined condition applied to the specimen preparing apparatus.

2. The system according to claim 1, wherein
the predetermined standard includes a lower limit and an upper limit.

3. The system according to claim 1, wherein
the predetermined condition is determined according to a type of the specimen preparing apparatus and/or a reagent used for preparing the specimen.

4. The system according to claim 1, wherein
the predetermined condition is determined according to the staining condition adopted in a testing facility.

5. The system according to claim 1, wherein
the quality control information is a chart displaying the feature value and the predetermined standard in a comparable manner or notification information displaying the feature value and the predetermined standard in a comparable manner.

6. The system according to claim 1, wherein
the predetermined standard is set by a user.

7. The system according to claim 1, wherein
the predetermined standard is set based on a guideline for the quality control.

8. The system according to claim 7, wherein
the predetermined standard is set based on the guideline that is determined according to a region in which the specimen is prepared.

9. The system according to claim 1, wherein
the predetermined standard is set based on information transmitted from a server.

10. The system according to claim 9, wherein
the information includes the predetermined standard.

11. The system according to claim 1, wherein
the specimen is prepared from the sample that includes cells collected from a subject.

12. The system according to claim 11, wherein
the specimen is a blood smear that is prepared from a blood sample collected from a subject.

13. The system according to claim 1, wherein
the predetermined standard includes at least color information of cells in the image data of the specimen.

14. The system according to claim 13, wherein
the cells are blood cells.
